# EUROPEAN PATENT SPECIFICATION

(11) **EP 1 846 441 B1**
(45) Date of publication and mention of the grant of the patent: **25.06.2008**
(21) Application number: 06706681.1
(22) Date of filing: 06.02.2006
(51) Int. Cl.: C07K 14/415, A61P 35/00, A61P 37/02

(54) **RIPROXIMIN , A NOVEL TYPE II RIBOSOME-INACTIVATING PROTEIN AND USES THEREOF**
RIPROXIMIN, EIN NEUES TYP II RIBOSOM-INAKTIVIERENDES PROTEIN UND SEINE VERWENDUNG
RIPROXIMIN, UNE PROTÉINE NOUVELLE INACTIVANT LES RIBOSOMES DE TYPE II ET SES UTILISATIONS

(30) Priority: 04.02.2005 EP 05002356
(43) Date of publication of application: 24.10.2007
(73) Proprietor: Deutsches Krebsforschungszentrum Stiftung des öffentlichen Rechts, 69120 Heidelberg (DE)
(72) Inventor: VOSS, Cristina, 69245 Bammental (DE); BERGER, Martin, 69126 Heidelberg (DE); SCHUMACHER, Carlos, 22391 Hamburg (DE)
(74) Representative: Schüssler, Andrea
(86) International application number: PCT/EP2006/001026
(87) International publication number: WO 2006/082098

(56) References cited:
- EP-A- 0 196 762
- EP-A- 0 335 476
- WO-A-00/66755
- DE-A1- 19 526 206
- US-B1- 6 803 358
- XU H. AND LIU W-Y: "Cinnamomin-a versatile Type II Ribosome-inativating Protein" ACTA BICHIMICA ET BIOPHYSICA SINICA, vol. 36, no. 3, 2004, pages 169-176, XP002338859 2004

### SEQUENCE LISTING

<110> Deutsches Krebsforschungszentrum Stiftung des öffentlichen
   Rechts
   voss, Cristina
   Berger, Martin
<120> Riproximin, a novel type II ribosome-inactivating protein and uses thereof
<130> K 3248
<150> EP 05002356.3
   <151> 2005-02-04
<160> 8
<170> PatentIn version 3.2
<210> 1
   <211> 1878
   <212> DNA
   <213> Ximenia americana
<400> 1
   cgcctggttg tggtggagaa ctggcttgrt cggaggacct gtttggcttg gcsagctaat      60
   caacatgccg actactacca aaccggtact ggaagaccca gttgtggtcg tcgatcatca     120
   ccaaaccacg ackcctrcag aaccaccaat tagtaccgat gcggttctaa tccacagatc     180
   tggacgaggg gamgaagaaa aagacagcat atccagtaca gtcctgggca tcgactatcc     240
   cacattcaga ttgagcctca gcggcctcac ccaaagagac tacaaccagt tcgtgagaga     300
   tctccgggat agattggcaa ggcacaatga ccggcggcac ggagtgccgg ttctgcgtca     360
   acccggcrcg gtgctcgaaa acgagcggtt tattttagtg gaactmacga acatggcaga     420
   ggatcrgacc atgacgtggg ccatagacgt caccaacctc tacttagtgg gttatcaagt     480
   cggaraccgt tcctacttct ttcgaggtgy cccggaggaa gcaacccgca atctcttcam     540
   cgacacaacg csccartcct acgatttcac cggcgactac acgcaactcc gacckcgcgc     600
   aggagttgga aayatagaca gtattaccct gggattcaac cagctagacg aatccatcaa     660
   ttacctgttc cgtcactccg gccaaaycca tacggaagaa gaaaagaata agcaggccac     720
   cgccatgata gtttgcattg tgatgattgc ggaggcggtg cgcttccggt acatccaggg     780
   aacgctggcc ggaaccatta ttggagacgg acgatayaga agctttaaca acaacggtgc     840
   tgtggccagg ttcataaaca cgtggcagga cctctcccaa gccatccagg aatccaacga     900
   agatggggtc tttcctaacc aaatccgatt gcagtckccy acttatgctg agtattgggc     960
   gaccaacgtg atgcccaatt tagttgcggc cattgggatc atgwtatact tctgtgcaaa    1020
   gcccccyccg cctcwatctg tcgccagtac cgttggttat aacggccaaa rttgtgcyga    1080
   tccacagcca acggtccgca tcagcggtcg agatggaaag tgtgccgatg tgaagggcgg    1140
   atactacaac gatggagacc caataatctt gtggccgtgt aaatccaaca ccgatgcgaa    1200
   tcaactgtgg atcttgaaga aagacgggac tattcgatcc aagggaaagt gcttgagcac    1260
   ttcgtttggc cgataccatt tcatctccac cgtcatgatc tacgactgca gcatagcgac    1320
   aaaggccgcc acgcgttgga aagtgtggga taatggaacg atcataaatt ctgcatcctc    1380
   gcttgttttg actkcggagt tgggaacgag cgggacgccg ctcactgtga gggccaattc    1440
   gtactccacg agccaaagtt ggcttccttc caatttttcg gagccttttg tagtgagttc    1500
   yattctgggg ctcaaagatc tctgcctaca agcaactgga aatggggtgt ggctggagca    1560
   atgtgtgacg aacaaggcgg agcagaagtg ggctctcttt ccagatcgtt caatacggcc    1620
   ttattcagat cgagatcttt gcctctcttg taatactgat gaaacttccc atcaattgaa    1680
   catcgtcaat cttcttagct gtggttctgc gtcgcctggt caacactgga tgttcagaaa    1740
   taatggcgat gttttaaaca tatactctgg rttggttatg gatgtgaaag rttccaatcc    1800
   aagccttcac caaatacttg cgtggccgcc taacggaggt cgtaaccaaa aatggatggt    1860
   ggttgtctga catgcaca                                                 1878
<210> 2
   <211> 1590
   <212> DNA
   <213> Ximenia americana
<400> 2
   accaattcgt gagagatytc agggatagat tggcaaggca caatgaccgg cggcacggag     60
   tgccggttct gcgtcaaccc ggcacggtgc ccgaaaacga gcggtttatt ttagtggaac    120
   tcacgaacat ggcagaggat cggaccatga cgtgggccat agacgtcacc ratctctact    180
   tggtgggtta tcgagttggg gacttttcct acttctttcg aggtgccccg gaggaagcaa    240
   ccggcaatct cttcaccggt acaacgcccc aatcctacga tttcaccggc gactacacgc    300
   aactccgacc gcgcgcagga gttggaaaca tagacagtat taccctggga ttcaaccagc    360
   tagacgaatc catcaattac ctgttccgtc actccggcca aacccatacg gaagaagaaa    420
   agaataagca ggccaccgcc atgatagttt gcattgtgat gattgcggag gcggtgcgct    480
   tccggtacat ccagggaagg ctggccctaa ccattgaaga cggacgatat ggaagcttta    540
   acggcracgg tgctgtggcc aggttcataa acacgtggca ggrcctctct caagccatcc    600
   aggaatccaa cgaagatggg gtctttccta accaaatccg attgcagtcc ccaacttatg    660
   ctgagtattg ggtgaccaac gtgatgccca atttagttgc ggccattggg atcatgttat    720
   acttctgtgc aaaaccccca ccgcctcaat ctgtcgccag taccgttggt tataacggcc    780
   aaacttgtgc agatccacag ccaacrgtcc gcatcagcgg tcgagatgga aagtgtgccg    840
   atgttaaggg cggatactac aacgatggag acccaataat cttgtggccg tgtaaatcca    900
   acaccgatgc gaatcaactg tggaccttga agaaagacgg gactattcga tctaagggaa    960
   agtgcttgag cacttcgttt ggtcgatacc atttcatctc caccgtcatg atccacgact   1020
   gcagcatcgc gacaaaagct gccacgcgtt ggaaagtatg ggacaacgga acgatcataa   1080
   attccgcgtc gtcgctcgtt ttgactgcgg agttgggaac aagcgggacg ccgctcactg   1140
   tgagggccaa ttcgtactcc acgagccaaa gttggcttcc ttccaatggt tcggagcctt   1200
   ttgtagtgag ttctattccg gggctcaagg gtctctgctt acaagcaact ggaaatgggg   1260
   tgtggctgga gcaatgtgtg acgaacaagg cggagcagaa gtgggctctc tttccagrtc   1320
   gttcaatacg gccttattca gatcgagatc tttgcctctc ttgtaatact gatgaaactt   1380
   cccatcaatt gaacatcgtc aatcttctta gctgtggttc tgcgtcgcct ggtcaacact   1440
   ggatgttcag aaataatggc gatgttttaa acatatactc tggattggtt atggatgtga   1500
   aagattccaa tccaagcctt caccaaatac ttgcgtggcc gcctaacgga ggtcgtaacc   1560
   aaaaatggct ggtggttgtc tgacatgcac                                     1590
<210> 3
   <211> 601
   <212> PRT
   <213> Ximenia americana
<220>
   <221> VARIANT
   <222> (25)..(25)
   <223> Ala or Thr
<220>
   <221> VARIANT
   <222> (43)..(43)
   <223> Glu or Asp
<220>
   <221> VARIANT
   <222> (102)..(102)
   <223> Ala or Thr
<220>
   <221> VARIANT
   <222> (121)..(121)
   <223> Arg or Gln
<220>
   <221> VARIANT
   <222> (141)..(141)
   <223> Asp or Asn
<220>
   <221> VARIANT
   <222> (159)..(159)
   <223> Asn or Thr
<220>
   <221> VARIANT
   <222> (208)..(208)
   <223> Ile or Thr
<220>
   <221> VARIANT
   <222> (314)..(314)
   <223> Ile or Leu
<220>
   <221> VARIANT
   <222> (324)..(324)
   <223> Gln or Leu
<220>
   <221> VARIANT
   <222> (336)..(336)
   <223> Ser or Asn
<220>
   <221> VARIANT
   <222> (444)..(444)
   <223> Ala or ser
<400> 3
   Met Pro Thr Thr Thr Lys Pro Val Leu Glu Asp Pro Val Val Val Val
   1 5 10 15
   Asp His His Gln Thr Thr Thr Pro Ala Glu Pro Pro Ile Ser Thr Asp
   20 25 30
   Ala Val Leu Ile His Arg Ser Gly Arg Gly Glu Glu Glu Lys Asp Ser
   35 40 45
   Ile Ser Ser Thr Val Leu Gly Ile Asp Tyr Pro Thr Phe Arg Leu Ser
   50 55 60
   Leu Ser Gly Leu Thr Gln Arg Asp Tyr Asn Gln Phe Val Arg Asp Leu
   65 70 75 80
   Arg Asp Arg Leu Ala Arg His Asn Asp Arg Arg His Gly Val Pro Val
   85 90 95
   Leu Arg Gln Pro Gly Ala Val Leu Glu Asn Glu Arg Phe Ile Leu Val
   100 105 110
   Glu Leu Thr Asn Met Ala Glu Asp Arg Thr Met Thr Trp Ala Ile Asp
   115 120 125
   Val Thr Asn Leu Tyr Leu Val Gly Tyr Gln Val Gly Asp Arg Ser Tyr
   130 135 140
   Phe Phe Arg Gly Val Pro Glu Glu Ala Thr Arg Asn Leu Phe Asn Asp
   145 150 155 160
   Thr Thr Arg Gln Ser Tyr Asp Phe Thr Gly Asp Tyr Thr Gln Leu Arg
   165 170 175
   Pro Arg Ala Gly Val Gly Asn Ile Asp Ser Ile Thr Leu Gly Phe Asn
   180 185 190
   Gln Leu Asp Glu Ser Ile Asn Tyr Leu Phe Arg His Ser Gly Gln Ile
   195 200 205
   His Thr Glu Glu Glu Lys Asn Lys Gln Ala Thr Ala Met Ile Val Cys
   210 215 220
   Ile Val Met Ile Ala Glu Ala Val Arg Phe Arg Tyr Ile Gln Gly Thr
   225 230 235 240
   Leu Ala Gly Thr Ile Ile Gly Asp Gly Arg Tyr Arg Ser Phe Asn Asn
                   245                 250                 255
   Asn Gly Ala Val Ala Arg Phe Ile Asn Thr Trp Gln Asp Leu Ser Gln
               260                 265                 270
   Ala Ile Gln Glu Ser Asn Glu Asp Gly Val Phe Pro Asn Gln Ile Arg
           275                 280                 285
   Leu Gln Ser Pro Thr Tyr Ala Glu Tyr Trp Ala Thr Asn Val Met Pro
       290                 295                 300
   Asn Leu Val Ala Ala Ile Gly Ile Met Ile Tyr Phe Cys Ala Lys Pro
   305                 310                 315                 320
   Pro Pro Pro Gln Ser Val Ala Ser Thr Val Gly Tyr Asn Gly Gln Ser
                   325                 330                 335
   Cys Ala Asp Pro Gln Pro Thr Val Arg Ile Ser Gly Arg Asp Gly Lys
               340                 345                 350
   Cys Ala Asp Val Lys Gly Gly Tyr Tyr Asn Asp Gly Asp Pro Ile Ile
           355                 360                 365
   Leu Trp Pro Cys Lys Ser Asn Thr Asp Ala Asn Gln Leu Trp Ile Leu
       370                 375                 380
   Lys Lys Asp Gly Thr Ile Arg Ser Lys Gly Lys Cys Leu Ser Thr Ser
   385                 390                 395                 400
   Phe Gly Arg Tyr His Phe Ile Ser Thr Val Met Ile Tyr Asp Cys Ser
                   405                 410                 415
   Ile Ala Thr Lys Ala Ala Thr Arg Trp Lys Val Trp Asp Asn Gly Thr
               420                 425                 430
   Ile Ile Asn Ser Ala Ser Ser Leu Val Leu Thr Ala Glu Leu Gly Thr
           435                 440                 445
   Ser Gly Thr Pro Leu Thr Val Arg Ala Asn Ser Tyr Ser Thr Ser Gln
       450                 455                 460
   Ser Trp Leu Pro Ser Asn Phe Ser Glu Pro Phe Val Val Ser Ser Ile
   465                 470                 475                 480
   Leu Gly Leu Lys Asp Leu Cys Leu Gln Ala Thr Gly Asn Gly Val Trp
                   485                 490                 495
   Leu Glu Gln Cys Val Thr Asn Lys Ala Glu Gln Lys Trp Ala Leu Phe
               500                 505                 510
   Pro Asp Arg Ser Ile Arg Pro Tyr Ser Asp Arg Asp Leu Cys Leu Ser
           515                 520                 525
   Cys Asn Thr Asp Glu Thr Ser His Gln Leu Asn Ile Val Asn Leu Leu
       530                 535                 540
   Ser Cys Gly Ser Ala Ser Pro Gly Gln His Trp Met Phe Arg Asn Asn
   545                 550                 555                 560
   Gly Asp Val Leu Asn Ile Tyr Ser Gly Leu Val Met Asp Val Lys Gly
                   565                 570                 575
   Ser Asn Pro Ser Leu His Gln Ile Leu Ala Trp Pro Pro Asn Gly Gly
               580                 585                 590
   Arg Asn Gln Lys Trp Met Val Val Val
           595                 600
<210> 4
   <211> 526
   <212> PRT
   <213> Ximenia americana
<220>
   <221> VARIANT
   <222> (6)..(6)
   <223> Leu or Phe
<220>
   <221> VARIANT
   <222> (57)..(57)
   <223> Asn or Asp
<220>
   <221> VARIANT
   <222> (182)..(182)
   <223> Asn or Asp
<220>
   <221> VARIANT
   <222> (194)..(194)
   <223> Asp or Gly
<220>
   <221> VARIANT
   <222> (439)..(439)
   <223> Asp or Gly
<400> 4
   Gln Phe Val Arg Asp Leu Arg Asp Arg Leu Ala Arg His Asn Asp Arg
   1               5                   10                  15
   Arg His Gly Val Pro Val Leu Arg Gln Pro Gly Thr Val Pro Glu Asn
               20                  25                  30
   Glu Arg Phe Ile Leu Val Glu Leu Thr Asn Met Ala Glu Asp Arg Thr
               35                  40                  45
   Met Thr Trp Ala Ile Asp Val Thr Asn Leu Tyr Leu Val Gly Tyr Arg
       50                  55                  60
   Val Gly Asp Phe Ser Tyr Phe Phe Arg Gly Ala Pro Glu Glu Ala Thr
   65                  70                  75                  80
   Gly Asn Leu Phe Thr Gly Thr Thr Pro Gln Ser Tyr Asp Phe Thr Gly
                   85                  90                  95
   Asp Tyr Thr Gln Leu Arg Pro Arg Ala Gly Val Gly Asn Ile Asp Ser
               100                 105                 110
   Ile Thr Leu Gly Phe Asn Gln Leu Asp Glu Ser Ile Asn Tyr Leu Phe
           115                 120                 125
   Arg His Ser Gly Gln Thr His Thr Glu Glu Glu Lys Asn Lys Gln Ala
       130                 135                 140
   Thr Ala Met Ile Val Cys Ile Val Met Ile Ala Glu Ala Val Arg Phe
   145                 150                 155                 160
   Arg Tyr Ile Gln Gly Arg Leu Ala Leu Thr Ile Glu Asp Gly Arg Tyr
                   165                 170                 175
   Gly Ser Phe Asn Gly Asn Gly Ala Val Ala Arg Phe Ile Asn Thr Trp
               180                 185                 190
   Gln Asp Leu Ser Gln Ala Ile Gln Glu Ser Asn Glu Asp Gly Val Phe
           195                 200                 205
   Pro Asn Gln Ile Arg Leu Gln Ser Pro Thr Tyr Ala Glu Tyr Trp Val
       210                 215                 220
   Thr Asn Val Met Pro Asn Leu Val Ala Ala Ile Gly Ile Met Leu Tyr
   225                 230                 235                 240
   Phe Cys Ala Lys Pro Pro Pro Pro Gln Ser Val Ala Ser Thr Val Gly
                   245                 250                 255
   Tyr Asn Gly Gln Thr Cys Ala Asp Pro Gln Pro Thr Val Arg Ile Ser
               260                 265                 270
   Gly Arg Asp Gly Lys Cys Ala Asp Val Lys Gly Gly Tyr Tyr Asn Asp
           275                 280                 285
   Gly Asp Pro Ile Ile Leu Trp Pro Cys Lys Ser Asn Thr Asp Ala Asn
       290                 295                 300
   Gln Leu Trp Thr Leu Lys Lys Asp Gly Thr Ile Arg Ser Lys Gly Lys
   305                 310                 315                 320
   Cys Leu Ser Thr Ser Phe Gly Arg Tyr His Phe Ile Ser Thr Val Met
                   325                 330                 335
   Ile His Asp Cys Ser Ile Ala Thr Lys Ala Ala Thr Arg Trp Lys Val
               340                 345                 350
   Trp Asp Asn Gly Thr Ile Ile Asn Ser Ala Ser Ser Leu Val Leu Thr
           355                 360                 365
   Ala Glu Leu Gly Thr Ser Gly Thr Pro Leu Thr Val Arg Ala Asn Ser
       370                 375                 380
   Tyr Ser Thr Ser Gln Ser Trp Leu Pro Ser Asn Gly Ser Glu Pro Phe
   385                 390                 395                 400
   Val Val Ser Ser Ile Pro Gly Leu Lys Gly Leu Cys Leu Gln Ala Thr
                   405                 410                 415
   Gly Asn Gly Val Trp Leu Glu Gln Cys Val Thr Asn Lys Ala Glu Gln
               420                 425                 430
   Lys Trp Ala Leu Phe Pro Asp Arg Ser Ile Arg Pro Tyr Ser Asp Arg
           435                 440                 445
   Asp Leu Cys Leu Ser Cys Asn Thr Asp Glu Thr Ser His Gln Leu Asn
       450                 455                 460
   Ile Val Asn Leu Leu Ser Cys Gly Ser Ala Ser Pro Gly Gln His Trp
   465                 470                 475                 480
   Met Phe Arg Asn Asn Gly Asp Val Leu Asn Ile Tyr Ser Gly Leu Val
                   485                 490                 495
   Met Asp Val Lys Asp Ser Asn Pro Ser Leu His Gln Ile Leu Ala Trp
               500                 505                 510
   Pro Pro Asn Gly Gly Arg Asn Gln Lys Trp Leu Val Val Val
           515                 520                 525
<210> 5
   <211> 493
   <212> DNA
   <213> Ximenia americana
<220>
   <221> misc_feature
   <222> (475)..(475)
   <223> n is a, c, g, or t
<220>
   <221> misc_feature
   <222> (485)..(485)
   <223> n is a, c, g, or t
<400> 5
   gagaacggca tggtcggagg acctgcttgg ctgcgccaac caccaagtag aaccgatggc 60
   gtcggtgata aatcagtgct acgcctttat tatcccaagc tgggacttgg caccctcggc 120
   ctcaccaaaa gagattacaa tcagttcgtg aaagatctgg gaagcagagt cgcaaatccg 180
   gatgacacgc ggcacggaat acccgtgctg cgtcagcccg gcacggtgcc agaagcccag 240
   cggtttattc tggtggagct aaccaacatg gcggaggagt ccgtgacagt ggccatcgat 300
   gtaaccgatc tgtacttggt cggttatcga gtcggagacc gttcgtactt ctttcgcggt 360
   gctccggagg aagcaacgcg cctccttttc accgatacac agcaacagac cttcgatttc 420
   accggccact accggccact cctgcgacga gcgcatctgc ggaacttaga cagtnttacc 480
   ctggnattca aca 493
<210> 6
   <211> 164
   <212> PRT
   <213> Ximenia americana
<220>
   <221> misc_feature
   <222> (159)..(159)
   <223> Xaa can be any naturally occurring amino acid
<220>
   <221> misc_feature
   <222> (162)..(162)
   <223> Xaa can be any naturally occurring amino acid
<400> 6
   Glu Asn Gly Met Val Gly Gly Pro Ala Trp Leu Arg Gln Pro Pro Ser
   1               5                   10                  15
   Arg Thr Asp Gly Val Gly Asp Lys Ser Val Leu Arg Leu Tyr Tyr Pro
               20                  25                  30
   Lys Leu Gly Leu Gly Thr Leu Gly Leu Thr Lys Arg Asp Tyr Asn Gln
           35                  40                  45
   Phe Val Lys Asp Leu Gly Ser Arg Val Ala Asn Pro Asp Asp Thr Arg
       50                  55                  60
   His Gly Ile Pro Val Leu Arg Gln Pro Gly Thr Val Pro Glu Ala Gln
   65                  70                  75                  80
   Arg Phe Ile Leu Val Glu Leu Thr Asn Met Ala Glu Glu Ser Val Thr
                   85                  90                  95
   Val Ala Ile Asp Val Thr Asp Leu Tyr Leu Val Gly Tyr Arg Val Gly
               100                 105                 110
   Asp Arg Ser Tyr Phe Phe Arg Gly Ala Pro Glu Glu Ala Thr Arg Leu
           115                 120                 125
   Leu Phe Thr Asp Thr Gln Gln Gln Thr Phe Asp Phe Thr Gly His Tyr
       130                 135                 140
   Arg Pro Leu Leu Arg Arg Ala His Leu Arg Asn Leu Asp Ser Xaa Thr
   145                 150                 155                 160
   Leu Xaa Phe Asn
<210> 7
   <211> 576
   <212> PRT
   <213> Ricinus communis
<400> 7
   Met Lys Pro Gly Gly Asn Thr Ile Val Ile Trp Met Tyr Ala Val Ala
   1               5                   10                  15
   Thr Trp Leu Cys Phe Gly Ser Thr Ser Gly Trp Ser Phe Thr Leu Glu
               20                  25                  30
   Asp Asn Asn Ile Phe Pro Lys Gln Tyr Pro Ile Ile Asn Phe Thr Thr
           35                  40                  45
   Ala Gly Ala Thr Val Gln Ser Tyr Thr Asn Phe Ile Arg Ala Val Arg
       50                  55                  60
   Gly Arg Leu Thr Thr Gly Ala Asp Val Arg His Glu Ile Pro Val Leu
   65                  70                  75                  80
   Pro Asn Arg Val Gly Leu Pro Ile Asn Gln Arg Phe Ile Leu Val Glu
                   85                  90                  95
   Leu Ser Asn His Ala Glu Leu Ser Val Thr Leu Ala Leu Asp Val Thr
               100                 105                 110
   Asn Ala Tyr Val Val Gly Tyr Arg Ala Gly Asn Ser Ala Tyr Phe Phe
           115                 120                 125
   His Pro Asp Asn Gln Glu Asp Ala Glu Ala Ile Thr His Leu Phe Thr
       130                 135                 140
   Asp Val Gln Asn Arg Tyr Thr Phe Ala Phe Gly Gly Asn Tyr Asp Arg
   145                 150                 155                 160
   Leu Glu Gln Leu Ala Gly Asn Leu Arg Glu Asn Ile Glu Leu Gly Asn
                   165                 170                 175
   Gly Pro Leu Glu Glu Ala Ile Ser Ala Leu Tyr Tyr Tyr Ser Thr Gly
   180 185 190
   Gly Thr Gln Leu Pro Thr Leu Ala Arg Ser Phe Ile Ile Cys Ile Gln
   195 200 205
   Met Ile Ser Glu Ala Ala Arg Phe Gln Tyr Ile Glu Gly Glu Met Arg
   210 215 220
   Thr Arg Ile Arg Tyr Asn Arg Arg Ser Ala Pro Asp Pro Ser Val Ile
   225 230 235 240
   Thr Leu Glu Asn Ser Trp Gly Arg Leu Ser Thr Ala Ile Gln Glu Ser
   245 250 255
   Asn Gln Gly Ala Phe Ala Ser Pro Ile Gln Leu Gln Arg Arg Asn Gly
   260 265 270
   Ser Lys Phe Ser Val Tyr Asp Val Ser Ile Leu Ile Pro Ile Ile Ala
   275 280 285
   Leu Met Val Tyr Arg Cys Ala Pro Pro Pro Ser Ser Gln Phe Ser Leu
   290 295 300
   Leu Ile Arg Pro Val Val Pro Asn Phe Asn Ala Asp Val Cys Met Asp
   305 310 315 320
   Pro Glu Pro Ile Val Arg Ile Val Gly Arg Asn Gly Leu Cys Val Asp
   325 330 335
   Val Arg Asp Gly Arg Phe His Asn Gly Asn Ala Ile Gln Leu Trp Pro
   340 345 350
   Cys Lys Ser Asn Thr Asp Ala Asn Gln Leu Trp Thr Leu Lys Arg Asp
   355 360 365
   Asn Thr Ile Arg Ser Asn Gly Lys Cys Leu Thr Thr Tyr Gly Tyr Ser
   370 375 380
   Pro Gly Val Tyr Val Met Ile Tyr Asp Cys Asn Thr Ala Ala Thr Asp
   385 390 395 400
   Ala Thr Arg Trp Gln Ile Trp Asp Asn Gly Thr Ile Ile Asn Pro Arg
   405 410 415
   Ser Ser Leu Val Leu Ala Ala Thr Ser Gly Asn Ser Gly Thr Thr Leu
   420 425 430
   Thr Val Gln Thr Asn Ile Tyr Ala Val Ser Gln Gly Trp Leu Pro Thr
   435 440 445
   Asn Asn Thr Gln Pro Phe Val Thr Thr Ile Val Gly Leu Tyr Gly Leu
       450                 455                 460
   Cys Leu Gln Ala Asn Ser Gly Gln Val Trp Ile Glu Asp Cys Ser Ser
   465                 470                 475                 480
   Glu Lys Ala Glu Gln Gln Trp Ala Leu Tyr Ala Asp Gly Ser Ile Arg
                   485                 490                 495
   Pro Gln Gln Asn Arg Asp Asn Cys Leu Thr Ser Asp Ser Asn Ile Arg
               500                 505                 510
   Glu Thr Val Val Lys Ile Leu Ser Cys Gly Pro Ala Ser Ser Gly Gln
           515                 520                 525
   Arg Trp Met Phe Lys Asn Asp Gly Thr Ile Leu Asn Leu Tyr Ser Gly
       530                 535                 540
   Leu Val Leu Asp Val Arg Ala Ser Asp Pro Ser Leu Lys Gln Ile Ile
   545                 550                 555                 560
   Leu Tyr Pro Leu His Gly Asp Pro Asn Gln Ile Trp Leu Pro Leu Phe
                   565                 570                 575
<210> 8
   <211> 531
   <212> PRT
   <213> viscum album
<400> 8
   Tyr Glu Arg Leu Arg Leu Arg Val Thr His Gln Thr Thr Gly Glu Glu
   1 5 10 15
   Tyr Phe Arg Phe Ile Thr Leu Leu Arg Asp Tyr Val Ser Ser Gly Ser
   20 25 30
   Phe Ser Asn Glu Ile Pro Leu Leu Arg Gln Ser Thr Ile Pro Val Ser
   35 40 45
   Asp Ala Gln Arg Phe Val Leu Val Glu Leu Thr Asn Gln Gly Gln Asp
   50 55 60
   Ser Ile Thr Ala Ala Ile Asp Val Thr Asn Ala Tyr Val Val Ala Tyr
   65 70 75 80
   Gln Ala Gly Asp Gln Ser Tyr Phe Leu Arg Asp Gly Ala Glu Thr His
   85 90 95
   Leu Phe Thr Gly Thr Thr Arg Ser Ser Leu Pro Ala Pro Arg Phe Asn
   100 105 110
   Gly Ser Tyr Pro Asp Leu Glu Arg Tyr Ala Gly His Arg Asp Gln Ile
   115 120 125
   Pro Leu Gly Ile Asp Gln Leu Ile Gln Ser Val Thr Ala Leu Arg Phe
   130 135 140
   Pro Gly Gly Ser Thr Arg Thr Gln Ala Arg Ser Ile Leu Ile Leu Ile
   145 150 155 160
   Gln Met Ile Ser Glu Ala Ala Arg Phe Asn Pro Ile Leu Trp Arg Tyr
   165 170 175
   Arg Gln Tyr Ile Asn Ser Gly Ala Ser Phe Leu Pro Asp Val Tyr Met
   180 185 190
   Leu Glu Leu Glu Thr Ser Trp Gly Gln Gln Ser Thr Gln Val Gln His
   195 200 205
   Ser Thr Asp Gly Val Phe Asn Asn Pro Ile Arg Leu Ala Ile Pro Pro
   210 215 220
   Gly Asn Phe Val Thr Leu Thr Asn Val Arg Asp Val Ile Ala Ser Leu
   225 230 235 240
   Ala Ile Met Leu Phe Val Cys Gly Glu Arg Pro Ser Ser Ser Asp Val
   245 250 255
   Arg Tyr Trp Pro Leu Val Ile Arg Pro Val Ile Ala Asp Asp Val Thr
   260 265 270
   Cys Ser Ala Ser Glu Pro Thr Val Arg Ile Val Gly Arg Asn Gly Met
   275 280 285
   Cys Val Asp Val Arg Asp Asp Asp Phe His Asp Gly Asn Gln Ile Gln
   290 295 300
   Leu Trp Pro Ser Lys Ser Asn Asn Asp Pro Asn Gln Leu Trp Thr Ile
   305 310 315 320
   Lys Arg Asp Gly Thr Ile Arg Ser Asn Gly Ser Cys Leu Thr Thr Tyr
   325 330 335
   Gly Tyr Thr Ala Gly Val Tyr Val Met Ile Phe Asp Cys Asn Thr Ala
   340 345 350
   Val Arg Glu Ala Thr Ile Trp Gln Ile Trp Gly Asn Gly Thr Ile Ile
   355 360 365
   Asn Pro Arg Ser Asn Leu Ala Leu Ala Ala Ser Ser Gly Ile Lys Gly
   370 375 380
   Thr Thr Leu Thr Val Gln Thr Leu Asp Tyr Thr Leu Gly Gln Gly Trp
   385 390 395 400
   Leu Ala Gly Asn Asp Thr Ala Pro Arg Glu Val Thr Ile Tyr Gly Phe
   405 410 415
   Arg Asp Leu Cys Met Glu Ser Asn Gly Gly Ser Val Trp Val Glu Thr
   420 425 430
   Cys Val Ser Ser Gln Gln Asn Gln Arg Trp Ala Leu Tyr Gly Asp Gly
   435 440 445
   Ser Ile Arg Pro Lys Gln Asn Gln Asp Gln Cys Leu Thr Cys Gly Arg
   450 455 460
   Asp Ser Val Ser Thr Val Ile Asn Ile Val Ser Cys Ser Ala Gly Ser
   465 470 475 480
   Ser Gly Gln Arg Trp Val Phe Thr Asn Glu Gly Ala Ile Leu Asn Leu
   485 490 495
   Lys Asn Gly Leu Ala Met Asp Val Lys Gly Ser Asn Pro Lys Leu Arg
   500 505 510
   Gln Ile Ile Ile Tyr Pro Ala Thr Gly Lys Pro Asn Gln Met Trp Leu
   515 520 525
   Pro Val Pro
   530

## Description

The present invention relates to a novel type II ribosome-inactivating protein, riproximin, as well as nucleic acid molecules encoding said protein. Furthermore, the present invention relates to therapeutic uses of riproximin.

Plant ribosome-inactivating proteins (RIPs) are a group of plant proteins with RNA-glycosidase activity which act on eukaryotic and prokariotic ribosomes. While some RIPs (e.g. the type II RIPs ricin, abrin, volkesin, viscumin) are highly toxic to humans and higher animals, other members of this class show only a low or no toxicity (e.g. the type II RIPs ebulin, nigrin, cinnamomin as well as all known type I and type III RIPs). The molecular mechanism of action of RNA N-glycosidase is to remove a specific adenine from a highly conserved loop ("sarcin/ricin domain") in the largest ribosomal RNA that is responsible for the interaction of both eukaryotic and prokaryotic elongation factors with the ribosome, thereby inhibiting protein synthesis. RIPs are classified into three types based on their primary structure. Type I RIP consists of a single, intact polypeptide of about 30kDa which, in some cases, is processed proteolytically into two shorter polypeptides held together by non-covalent interactions. Type II RIP is composed of two polypeptide chains, termed A- and B-chain, being held together by a disulphide bridge. The N-terminal A-chain comparable with type I RIP having the function of a RNA N-glycosidase being able to inhibit protein synthesis in a cell free-lysate is linked by a disulphide bridge to the C-terminal B-chain that is a lectin having carbohydrate-binding activity. The toxic effects of type II RIPs are based on a mechanism involving cellular uptake of the protein mediated by the binding of the B-chain to sugar moieties on the cell surface, followed by an internalization of the A-chain, which inactivates the ribosomes and thus terminsates proteins synthesis (Lord et al. (2003) *Toxicol. Rev.22,* 53-64). Type III RIP consists of a type I RIP-like N-terminal domain and a C-terminal domain of unknown function.

Many studies have been performed on the applications of RIPs in drug development, immuno-modulation and crop-plant biotechnology due to their toxicity to viruses, tumour cells, insects and plant fungal pathogens. RIPs have also been used as a powerful probe to study the structure of ribosomes. However, the physiological function that RIPs play in the plant cell is unclear. Previous studies revealed that many RIPs are involved in defence mechanisms in plant cells and terminate protein synthesis under appropriate physiological conditions and thus are involved in metabolic regulation. RIPs occur in various plant tissues but are most prominent in storage organs like cotyledon, endosperm, bark, tubers, storage roots, bulbs and rhizomes. Apart from a few exceptions, RIPs occur as mixtures of more or less closely related isoforms; see van Damme et al., Crit.Rev.Plant Sci. 20 (2001), 395-465.

One member of the type II RIP family is viscumin, a toxic lectin from mistletoe. It was demonstrated that viscumin is remarkably similar in structure and mechanism of action to the plant toxins abrin, ricin, and modeccin and that one of the constituent peptide chains of viscumin, the A chain, inhibits cell-free protein synthesis by inactivating catalytically the large ribosomal subunit. Moreover, viscumin seems to be a promising drug candidate for cancer therapy and phase I/II clinical trials are currently carried out. Thus, there is a need for the isolation and characterization of further members of the type II RIP family which might be useful for cancer therapy.

Thus, the technical problem underlying the present invention is to provide alternative type II RIPs which might be useful for therapy of tumors, modulation of the immune system, antiviral treatment, protection against insects and plant fungal pathogens.

The solution to said technical problem is achieved by providing the embodiments characterized in the claims. During the experiments resulting in the present invention a protein from *Ximenia americana* could be isolated and characterized. This protein, which was named riproximin, is a member of the type II RIPs and shows antineoplastic activities, thus is useful, for example, for therapy of tumours, modulation of the immune system, antiviral treatment, protection against insects and plant fungal pathogens.

### BRIEF DESCRIPTION OF THE DRAWINGS

Figure 1: *In vitro* comparison of the anti-neoplastic activity of an aqueous extract from *Ximenia Americana* containing riproximin in 16 human and one rodent cell lines
   Extract concentrations describing IC₅₀ values are given in µg crude extract/ml, i.e. the amount (µg) of dry *Ximenia ameri*cana powder used for the preparation of the respective dilution. The average IC₅₀ value of all cell lines was 49 µg crude extract/ml medium.
Figure 2: *In vivo* antitumor activity of an aqueous extract containing riproximin, following tannin pre-extraction from *Ximenia Americana*
   The effect of the aqueous extract containing riproximin, following tannin pre-extraction, was analyzed by the CC531 colon carcinoma liver metastasis model (Wittmer et al., Clin.Exp.Met. 17 (1999), 369-376). The maximum effect of the antimetabolite Alimta is given for comparison. Extract concentrations are given in mg crude extract/kg body weight, i.e. the amount (mg) of dry *Ximenia americana* powder used for the preparation of the respective dilution.

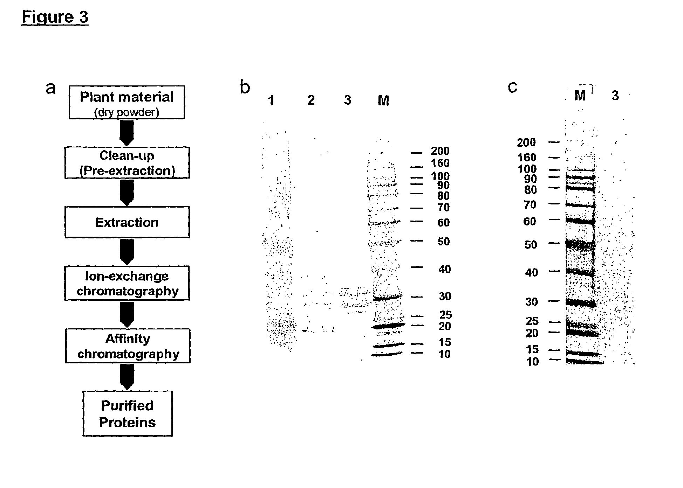

Figure 3: Purification of the anti-neoplastic protein, riproximin
   **(a)** Purification flow chart;
   **(b)** SDS-PAGE (under reducing conditions) of crude extract after tannin extraction (1), total protein fraction after ion-exchange chromatography (2), and affinity-chromatography purified proteins (3); M, molecular weight markers (MW in kDa).
   **(c)** SDS-PAGE (under non-reducing conditions) of affinity chromatography purified proteins (3); M, molecular weight markers (MW in kDa).

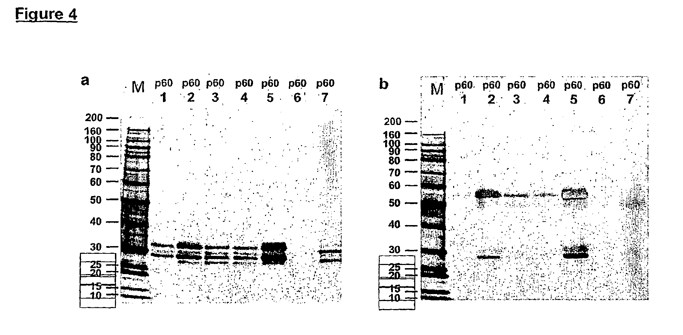

Figure 4: Various affinity-purified protein preparations
   Lanes 1-7: 7 different batches of riproximin were analyzed by reducing SDS-PAGE **(a)** or non-reducing SDS-PAGE **(b).** M = molecular weight markers (weight in kDa). Protein concentrations and IC₅₀ values are given in ng dry protein/ml. The amount (ng) of dry protein in the affinity purified fraction was determined by lyophilisation.

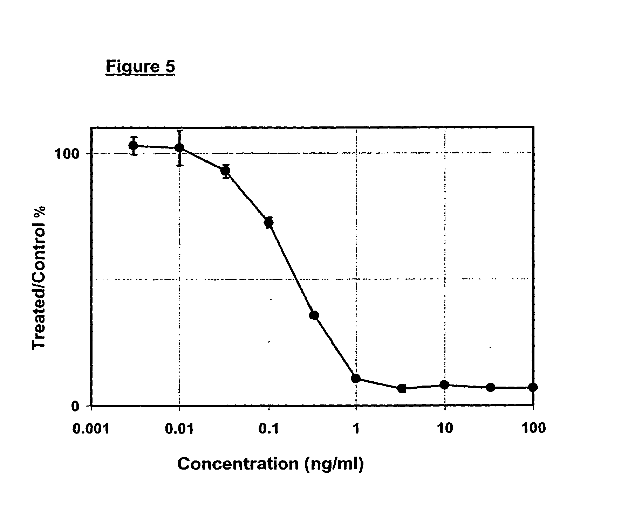

Figure 5: Cytotoxic activity of an affinity-purified riproximin preparation in MCF7 breast cancer cells
   The viable cell count was determined by the MTT-assay (Konstantinov et al., Int.J.Cancer 77 (1998), 778-86). The effect of increasing concentrations (ng/ml medium) is given in percent of untreated control.

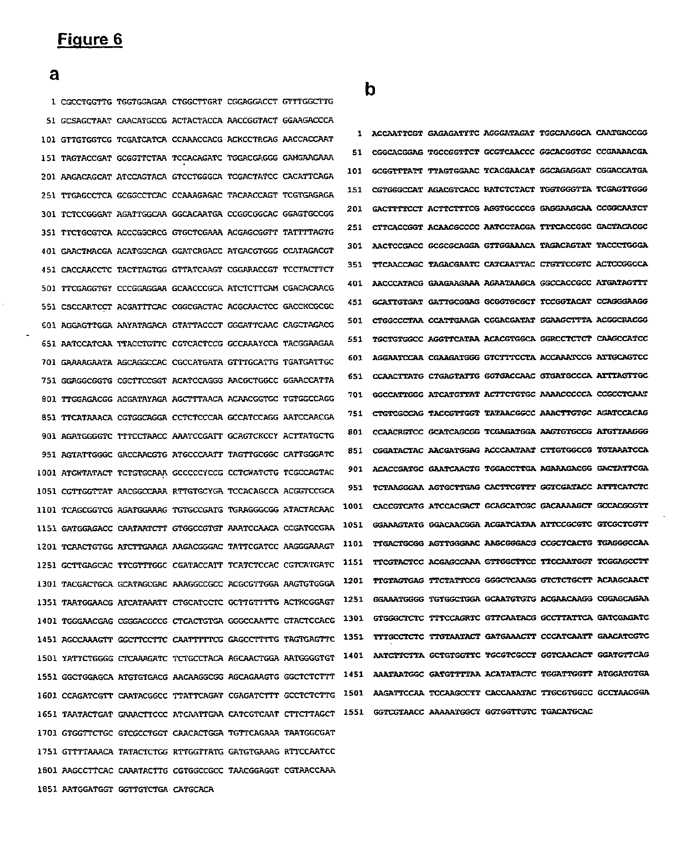

Figure 6: Nucleotide sequence of the riproximin encoding gene
   **(a)** complete cDNA sequence derived from *Ximenia americana* of US-origin (SEQ ID NO:1) .
   **(b)** incomplete coding genomic DNA sequence derived from *Ximenia americana of Tanzanian origin* (SEQ ID NO:2). About 250 bp are missing at the 5'end.

   The US- and Tanzanian riproximin DNA sequences share 95 - 97% identity, depending on the respective allelic state.

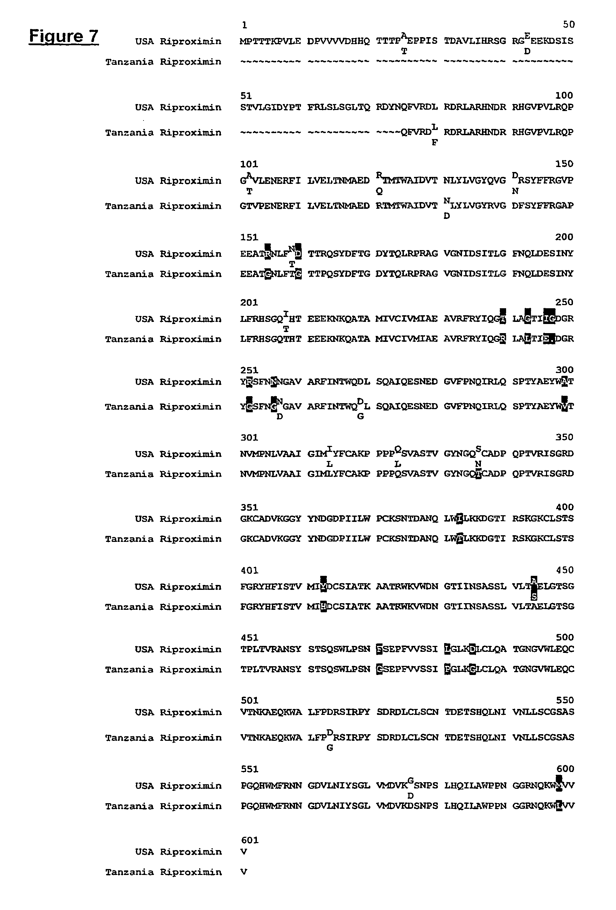

Figure 7: Derived amino acid sequence of riproximin
   The complete amino acid sequences of US-riproximin (SEQ ID NO:3) as well as an incomplete C-terminal sequence of the Tanzanian riproximin (SEQ ID NO:4) were aligned. Allelic amino acid variations are indicated by giving the respective abbreviations in super- and subscript, and light grey highlighted. Amino acids differing between the US and Tanzanian proteins are given in white and highlighted in black.
   Allelic riproximin amino acid sequences show 97.9% similarity and 97.8% identity. Riproximin amino acid sequences of US- and Tanzanian origin share 94.9 - 96.2% similarity and 94.3-95.8% identity, depending on the respective allelic state (percent identity=percent of identical aminoacids within the two sequences; percent similarity=percent identical and similar aminoacids (aminoacids with the same function) between the two sequences).

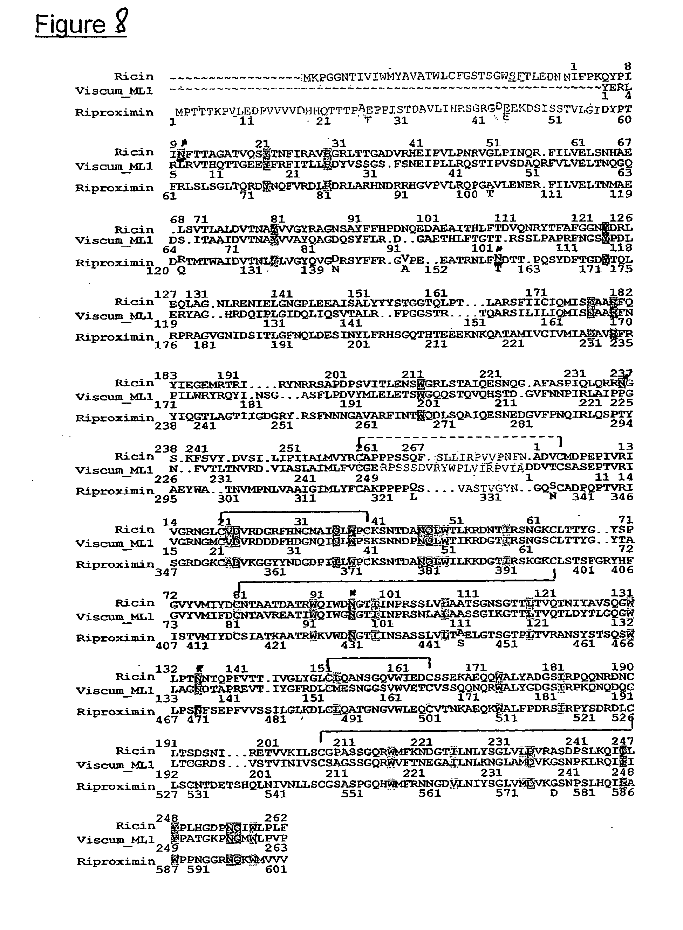

Figure 8 shows a protein sequence alignment of riproximin with ricin (acc. no. P02879) and viscum lectin I (acc. no. P81446). The sequences of the ricin and viscum I A and B-chains, as well as the sequence of the riproximin A and B-chains, are given in black, sequences of the N-terminal signal and of the linker peptides are given in grey. Cystein residues are highlighted, the positions of the intramolecular and intermolecular disulphide bonds are indicated by solid and dashed lines, respectively. The amino acids involved in the catalytically active center of the A-chain and the sugar binding sites of the B-chain are highlighted. Potential N-glycosylation sites are highlighted. Amino acids building the hydrophobic core of the B-chain are highlighted in grey.

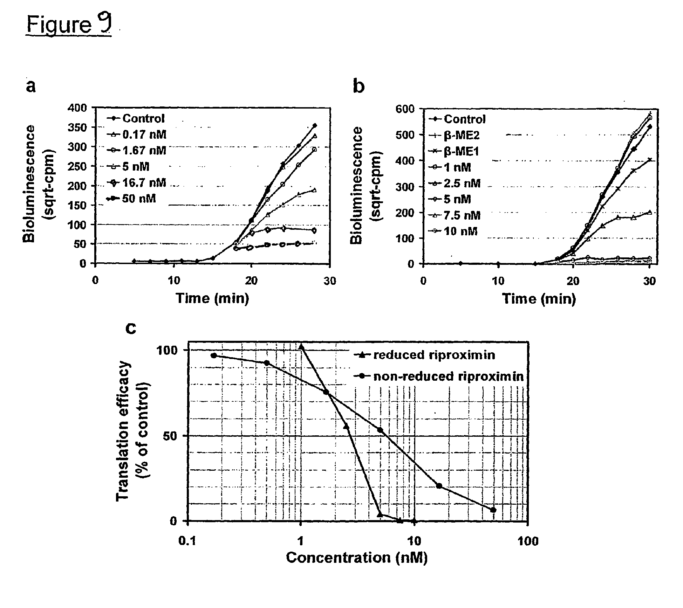

Figure 9: Effect of the affinity-purified protein sample in an *in vitro* translation assay. a- kinetics of luciferase synthesis in the presence of various concentrations of the non-re- duced proteins; b - kinetics of luciferase synthesis in the presence of various concentrations of the reduced proteins; c - comparison of the concentration-dependent translation efficacies in response to reduced and non-reduced proteins.

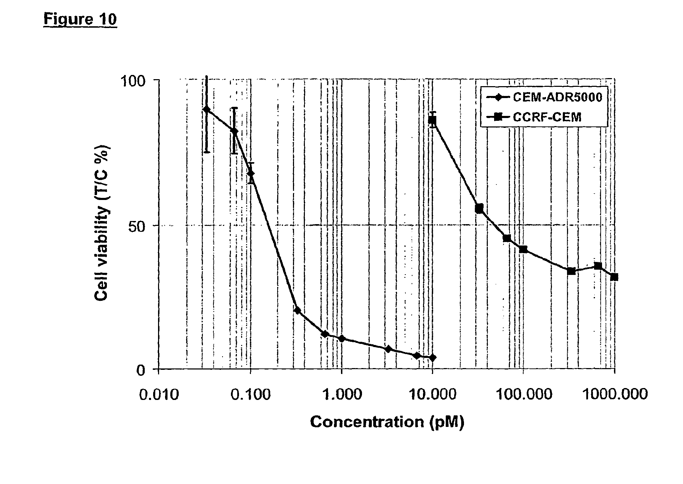

Figure 10: Cytotoxic effects of riproximin in CCRF-CEM wild-type and CEM-ADR5000 doxorubicin-resistant cells.

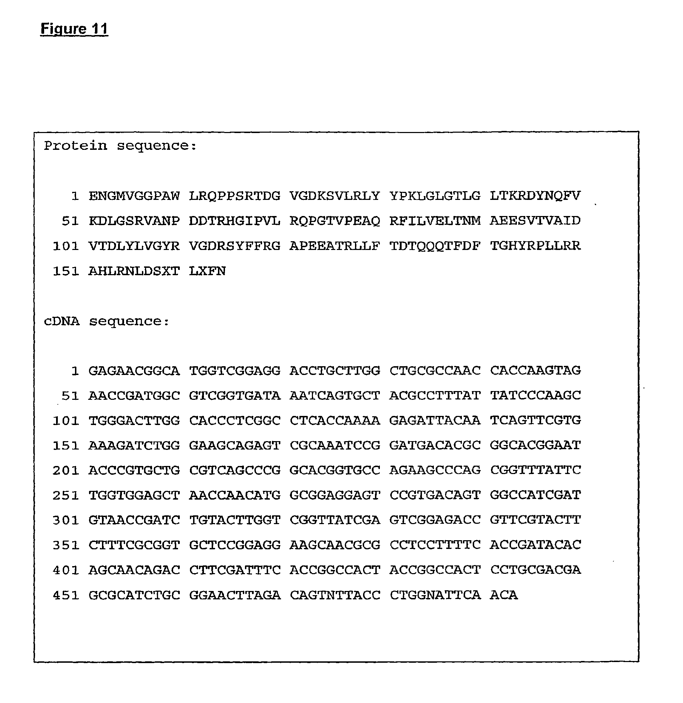

Figure 11: Protein (SEQ ID NO:6) and cDNA (SEQ ID NO:5) sequences of a riproximin-homolog (Rpx2) from *Ximenia americana.*

The protein and the sequences were obtained according to the methods described in Example 1.

Thus, the present invention relates to a nucleic acid molecule encoding the plant type II ribosome-inactivating protein (RIP) riproximin exhibiting anti-neoplastic activity or a polypeptide exhibiting a biological property of riproximin, which is:
(a) a nucleic acid molecule encoding a polypeptide that comprises the amino acid sequence as depicted in Figure 7 (SEQ ID NOs:3 and 4) or Figure 11 (SEQ ID NO:6);
(b) a nucleic acid molecule comprising the nucleotide sequence as depicted in Figure 6(a) (SEQ ID NO:1) or (b)(SEQ ID NO:2) or in Figure 11 (SEQ ID NO:5);
(c) a nucleic acid molecule encoding a polypeptide the sequence of which is at least 70%, preferably at least 80%, 85%, 90%, 95% or 98%, in the most preferred embodiment, 98% identical to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code; or
(e) a nucleic acid molecule, which represents a fragment or a variant of a nucleic acid molecule of (a) to (d).

As used herein, a polypeptide exhibiting a biological property of riproximin is understood to be a polypeptide having at least one of the biological activities of a type II RIP. These activities include cytotoxic activity *in vitro* and *in vivo,* immunomodulatory activity *in vivo,* inhibition of protein synthesis, specific hydrolysis of the A⁴³²⁴ in the ricin-sarcin loop of eukaryotic 28S rRNA, polynucleotide N-glycosylase activity on DNA or RNA, antiviral, antifungal and insecticide activity.

In a preferred embodiment of the invention the polypeptide is cytotoxic to drug resistant cells, particularly cells over-expressing MDR1 protein or another member of the ABC (ATP-binding cassette) transporter proteins.

Preferably, the polypeptide according to the invention is isolated and/or purified, i.e., substantially free of cellular material or culture medium when produced by recombinant DNA techniques.

In a first embodiment, the invention provides a nucleic acid molecule encoding a riproximin polypeptide that comprises the amino acid sequence as depicted in Figure 7 (SEQ ID NOs:3 or 4) or Figure 11 (SEQ ID NO:6).

The present invention also provides a nucleic acid molecule encoding an riproximin polypeptide comprising the nucleotide sequence as depicted for DNA in Figure 6(a) (SEQ ID NO:1) or (b) (SEQ ID NO:2) or Figure 11 (SEQ ID NO:5).

The nucleic acid molecules of the invention can be both DNA and RNA molecules. Suitable DNA molecules are, for example, genomic or cDNA molecules. It is understood that all nucleic acid molecules encoding all or a portion of riproximin are also included, as long as they encode a polypeptide with biological activity. The nucleic acid molecules of the invention can be isolated from natural sources or can be synthesized according to known methods.

The present invention also provides nucleic acid molecules encoding a polypeptide the amino acid sequence of which is at least 70%, preferably at least 80%, 85%, 90%, 95% or 98%, in the most preferred embodiment, 98% identical to the amino acid sequence of the polypeptide of Figure 7 (SEQ ID NOs:3 or 4) or of Figure 11 (SEQ ID NO:6). Such amino acid sequences are characterized by deletion, substitution and/or insertion of amino acid residue(s) compared to the amino acid sequences shown in Figure 7 (SEQ ID NOs:3 or 4) or in Figure 11 (SEQ ID NO:6) or are the result of recombination. They can be naturally occurring variations, for example sequences from other organisms, or mutations that can either occur naturally or that have been introduced by specific mutagenesis. They can also be isolated, e.g., from genomic or cDNA libraries that were produced from plant cells or tissues. In order to identify and isolate such nucleic acid molecules the molecules of the invention or parts of these molecules or the reverse complements of these molecules can be used, for example by means of hybridization. As a hybridization probe nucleic acid molecules can be used, for example, that have exactly or basically the nucleotide sequence as depicted in Figure 6 (SEQ ID NOs:1 or 2) or in Figure 11 (SEQ ID NO:5), respectively, or parts of these sequences. The fragments used as hybridization probe can be synthetic fragments that were produced by means of conventional synthetic methods and the sequence of which basically corresponds to the sequence of a nucleic acid molecule of the invention.

The nucleic acid molecules of the present invention also include molecules which differ from the nucleic acid molecules with sequences shown in Figure 6(a) (SEQ ID NO: 1) or (b) (SEQ ID NO:2) or in Figure 11 (SEQ ID NO:5) due to the degeneracy of the genetic code.

In a further embodiment, the present invention provides nucleic acid molecules which comprise fragments or variants of the nucleic acid molecules described above encoding a polypeptide of the invention which still show a biological activity. "Fragments" are understood to be stretches of DNA and are parts of the nucleic acid molecules that are long enough to encode one of the described polypeptides. In a particular embodiment of the invention the fragments may either comprise the A- and/or the B-chain of the protein. Furthermore, the fragments may encode truncations of the A- and/or B-chain of the protein which contain only a portion of the A chain which is necessary for exerting its cytotoxic effect, e.g., truncations where 10, 15, 20, 25 or 30 terminal amino acid residues are removed from the A chain which remains toxic activity; and/or which contain only a portion of the B-chain which is necessary for galactose recognition, cell binding and transport into the cell cytoplasm, e.g., truncations where 10, 15, 20, 25 or 30 terminal amino acid residues are removed from the B-chain which retains its activity. Fragments according to the invention also comprise nucleic acid molecules specifically hybridizing to (transcripts of) the nucleic acid molecules of the invention. These nucleic acid molecules can be used, for example, as (gene) probes or primers, e.g., for isolating homologous genes from related plants and, preferably, are oligonucleotides having a length of at least 10, in particular of at least 15 and particularly preferred of at least 50 nucleotides. The nucleic acid molecules and oligonucleotides of the invention can also be used, for example, as primers for a PCR reaction.

The variants can be either naturally occurring variants or synthetically produced variants or variants produced by recombinant DNA processes. Naturally occurring variants include variants between subspecies of *Ximenia americana* (e.g., X.a. of African or American origin), as well as isoenzymes occurring in the same species, with amino acid sequences showing more than 70% identity to the amino acid sequence depicted in Figure 7 (SEQ ID NOs:3 or 4) or in Figure 11 (SEQ ID NO:6).

Generally, by means of conventional molecular biological processes it is possible (see, e.g., Sambrook et al., supra) to introduce different mutations into the nucleic acid molecules of the invention. As a result, riproximin polypeptides or riproximin related polypeptides with possibly modified biological properties are synthesized. One possibility is the production of deletion mutants in which nucleic acid molecules are produced by continuous deletions from the 5'- or 3'-terminus of the coding DNA sequence and that lead to the synthesis of polypeptides that are shortened accordingly. Another possibility is the introduction of single-point mutation at positions where a modification of the amino acid sequence influences, e.g., the proteolytic properties. By this method muteins can be produced, for example, that possess a modified Kₘ-value or that are no longer subject to the regulation mechanisms that normally exist in the cell, e.g. with regard to allosteric regulation or covalent modification.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptides encoded by the various variants of the nucleic acid molecules of the invention show some of the common characteristics, such as anti-neoplastic activity, cytotoxic activity *in vitro* and *in vivo,* immunomodulatory activity *in vivo*, inhibition of protein synthesis, specific hydrolysis of the A⁴³²⁴ in the ricin-sarcin loop of eukaryotic 28S rRNA, polynucleotide N-glycosylase activity on DNA or RNA, antiviral, antifungal and insecticide activity, immunological reactivity or conformation, lectin activity, physical properties like the solubility or activity in drug-resistant tumor cells over expressing the MDR1 protein.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors or the invention. A host cell is understood to be an organism that is capable to take up in vitro recombinant DNA and, if the case may be, to synthesize the polypeptides encoded by the nucleic acid molecules of the invention. Preferably, these cells are prokaryotic or eukaryotic cells, for example mammalian cells, bacterial cells, insect cells, plant cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a polypeptide exhibiting a biological property of riproximin and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an anti-riproximin antibody, or, e.g., can be substantially purified by the one-step method described in Smith and Johnson, Gene 67; 31-40 (1988).

These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. The polypeptides are preferably in a substantially purified form. Furthermore, the polypeptides may be further processed, e.g by glycosylation. This glycosylation which may occur within or outside of the production cell may have an influence on the activity and behavior of the polypeptide.

Furthermore, the present invention provides conjugates comprising a protein of the present invention or an A-chain fragment thereof as partner. Such conjugates, preferably immunoconjugates, can be prepared according to methods well known to the person skilled in the art, e.g., by recombinant DNA technology and/or conventional chemical synthesis. Immunoconjugates are, e.g. suitable for reducing proliferation of neoplastic cells. For example, the binding partner of riproximin, i.e. antibody or part thereof as described below is chosen so as to be specificially reactive with one or more neoplastic cell specific internalizing antigens present on the surface of the neoplastic cell. The conjugate is then placed in contact with the neoplastic cells and allowed to bind the neoplastic cell specific internalizing antigen. Once bound, the riproximin or related protein will be internalized through the endo-cytic pathway. Thus, such conjugates are useful for a variety of therapies, e.g., for treatment of tumours or immunomodulation. A further example of a conjugate of the present invention is a riproximin-ferritin conjugate. The generation of ferritin conjugates is, e.g. described by Schultz et al., Biochemistry 20(12) (1981), 3412-8.

The present invention also relates to an antibody that binds specifically to a riproximin or a related polypeptide as defined above. The term "antibody", preferably, relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

For certain purposes, the antibody of the present invention can be detectably labeled, for example, with a radioisotope, a bioluminescent compound, a chemiluminescent compound, a fluorescent compound, a metal chelate, or an enzyme.

The present invention also relates to a pharmaceutical composition comprising a nucleic acid molecule, polypeptide, conjugate or recombinant vector according to the present invention and a pharmaceutically acceptable excipient, diluent or carrier. Preferably, the pharmaceutical composition is a steril composition.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by oral, intravenous, intraperitoneal, subcutaneous, intramuscular, topical, intradermal, intrarterial, intratumoral or extracorporal administration. The route of administration, of course, depends on the nature of the disease, e.g., tumor, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease (e.g. tumor), general health and other drugs being administered concurrently.

The delivery of the nucleic acid molecules of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, e.g., a tumor to be treated, the nucleic acid molecules of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to the use of the above compounds of the invention (nucleic acid molecules, proteins, conjugates, antibodies, vectors etc.) for the preparation of a pharmaceutical composition for treatment of a tumor or for immunomodulation. For such uses, plant extracts containing a protein or nucleic acid of the present invention are also suitable. Such extracts can be prepared according to the methods described in the examples. Preferably, the crude plant extract is at least partially purified, e.g., by aqueous extraction from the plant material of a plant from the genus of *Ximenia,* preferably of the species of *Ximenia americana.* The plant extract can be further purified, e.g., by pre-extraction of the plant material, preferably with 70% acetone in water. Additional preferred purification methods suitable for removing protein contaminants include ion exchange and/or affinity chromatography. Therefore, the present invention further relates to the use of a plant from the genus of *Ximenia* for obtaining a type II ribosome-inactivating protein, which exhibits at least one of the activities of a type II ribosome-inactivating protein such as cytotoxic activity, immunomodulatory activity *in vivo,* inhibition of proteins synthesis or polynulceotide N-glycosylase activity.

The following examples illustrate the invention.

### Example 1

### Characterization of the crude extract obtained from Ximenia americana

### (A) Preparation of crude extract from Ximenia americana

Dry plant material from *Ximenia americana* was powdered and an aqueous extract was prepared by suspending the powder in water. After a brief centrifugation, the supernatant was collected. For *in vitro* experiments the crude extract was diluted in culture medium and sterilized by filtration.

### (B) Biological effects

### (1) Cell cultures

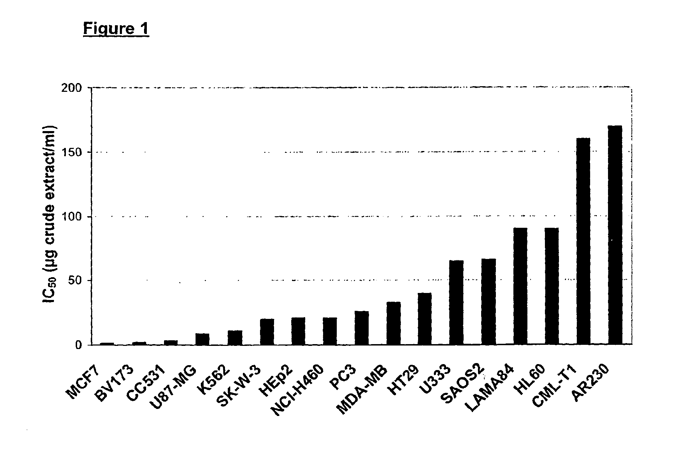

The effects of the aqueous extract on various tumor cell lines (16 human and 1 rodent cell lines) were investigated. The strongest growth inhibition was found for MCF7 breast cancer cells, BV173 chronic myeloid (CM) leukemia, astrocytomaglioblastoma U87-MG cells as well as the rat colon carcinoma cell line CC531. The results are summarized in Table 1 and Figure 1. Extract concentrations and IC₅₀ values are given in µg crude extract/ml, i.e. the amount (µg) of dry *Ximenia americana* powder used for the preparation of the respective dilution.

### (2) Animals

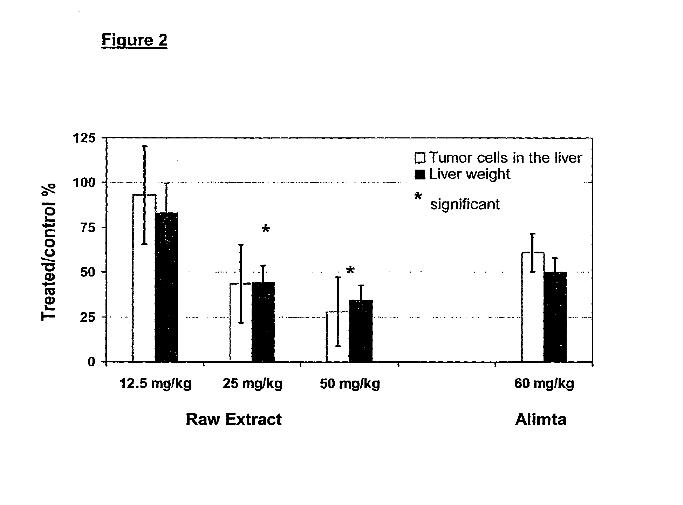

The effect of the aqueous extract *in vivo* was determined by use of the liver metastasis model of the isogenic colon carcinoma cell line CC531. Various doses of the aqueous extract after tannin pre-extraction were applied i.v. for five consecutive days (with day 1 being the day of implantation of tumor cells). The results are shown in Figure 2. For comparison, the maximum effect of the cytostatic compound Alimta in the same animal model is shown (* indicates a significant effect. The multiple nonparametric Kruskal-Wallis test (ADAM, DKFZ Heidelberg) was used to compare tumor cell numbers and liver weights of treated animals with controls; p-values<0,05 were considered significant.).

### (C) Physicochemical characterization of the extract/active agent

### (1) Solubility

Water soluble; PBS: strong growth inhibiting effect *in vitro* (IC₅₀= 1.8 µg crude extract/ml on MCF7 mamma carcinoma cells); only partially soluble in methanol: the growth inhibiting effect in vitro is lower (by a factor of 300; IC₅₀= 600 µg crude extract/ml on MCF7 mamma carcinoma cells); insoluble in ethanol, acetone, chloroform; can be well extracted with water/methanol mixtures (methanol < 30%; IC50 < 2µg crude extract/ml); can be poorly extracted with water/methanol mixtures (methanol > 70%; IC₅₀ > 333 µg crude extract/ml on MCF7 mamma carcinoma cells); the active compound can be completely extracted after pre-extraction of the powder with ethanol (IC₅₀ = 2 µg crude extract/ml on MCF7 mamma carcinoma cells); pre-extraction with methanol renders a second extraction with water less efficient (IC₅₀ = 14 µg crude extract/ml on MCF7 mamma carcinoma cells). A pre-extraction with 70% acetone in water renders the second extraction with water more efficient (IC₅₀ = 0,7 µg crude extract/ml on MCF7 cells).

### (2) Influence of pH on solubility/biological activity

Extraction with 10 mM buffer/acid/base-solution (pH 2 to 13; short-term exposure of one hour or less) did not show any negative effect. Diluted acids (10 mM HCl, 2 hours at RT) partially destroy the biological activity. Concentrated acids completely destroy the biological activity. Diluted bases (10 mM NaOH, 2 hours at RT) do not show any effect on biological activity, concentrated bases completely destroy the biological activity. Extract concentrations and IC₅₀ values are given in µg crude extract/ml, i.e. the amount (µg) of dry *Ximenia americana* powder used for the preparation of the respective dilution.

### (3) Precipitation with organic solvents

By addition of ethanol, methanol, acetone or acetonitril complete precipitation of the active compound from a aqeous extract can be achieved. The pellet can be completely re-solved (water or buffer) without any loss of biological activity.

### (4) Estimation of molecular weight by ultracentrifugation

The active compound completely passes a ultrafiltration membrane having a cut-off value of 100 kDa, partially passes a membrane having a cut-off value of 50 kDa and does not pass a membrane having a cut-off value of 10 kDa.

### (5) Test on tannins (polyphenoles)

The methanol extract contains high concentrations of polyphenoles. By use of C18 reverse phase HPLC/MS various catechines, gallocatechines and gallic acid were identified as components of the extract. A method for extracting tannins from the crude material was established. However, the tannin containing fraction did not show any effect *in vitro.* In the aqueous extract which had been prepared from the pre-extracted tannin-free crude material polyphenoles could not be identified by HPLC. *In vitro,* the effect of this extract was stronger compared to the effect of the extract which had been prepared from the tannin containing initial sample.

### (6) Results of digestion with trypsin, proteinase K, DNase and RNase

There was no loss of activity after treatment of the aqueous extract prepared from the initial sample for 24h at 37° with trypsin, proteinase K, RNase or DNase. However, the effect of the tannin-free extract was completely abolished after proteinase K treatment (but not after treatment with trypsin).

### (D) Stability of the biological activity

### (1) Stability of the Ximenia Americana dry powder and reproducibility

After dry storage of the powder at 4°C the biological activity was stable for more than three years. Samples of the powder that were assayed after 1, 2 or 3 years showed a practically constant effect *in vitro.*

### (2) Stability of the aqueous extract

Loss of activity up to 20% (after one day), 50% (after one weak) and 80% (after one month) when stored at 4°C was observed. If stored at -20°C for 1 year there was no loss of activity.

### (3) Temperature sensitivity of the aqueous extract

After storage at RT for 2 to 3 h no loss of activity was observed. Slight loss of activity was observed after 10 to 30 min at 50°. After incubation at 90°C for 10 min, the activity was completely destroyed.

### (4) Lyophilization

After lyophilization no loss of activity was observed. 97% of the lyophilized material can be re-disolved in water or PBS. The lyophylized material is stable over a period of one year if stored in the dry at 4°C or in solution at -20°C.

Conclusion: The stability properties of the extract show that the active compound is a protein.

### (5) Binding- and purification characteristics

C18 resins: aqueous extract, pH 7.0: no binding of the active compound. The methanol eluate of the column contains polyphenoles and catechines.
Cation exchange resins: No binding of the active compound at pH 7.0 in 20 mM Tris-HCl. No measurable purification,of the extract.
Anion exchange resins: At pH 7.0 in 20 mM Tris-HCl there is binding of the active compound to weak and strong anion exchange media. Active fractions can be eluted with 20 mM Tris-HCl, pH 7.0, with addition of 200 - 400 mM NaCl. Two different fractions can be partially separated. Components of the extract which strongly bind to the resin can only be eluted by NaOH.
Affinity binding: The active compound binds to partially hydrolyzed Sepharose, i.e., shows an affinity to galactose residues. After washing, an active fraction can be eluted from the material by use of a buffer containing 0.1 M galactose.

### (E) Purification of the active compound

The results are shown in Figure 3. The flow chart of the purification of the active compound is given in Figure 3a. The achieved purification grade was monitored by SDS-gel electrophoresis (Figure 3b). After a pre-extraction with an admixture of 70% acetone in water, the plant powder was pelleted by centrifugation and air-dried. A second extraction was performed by suspending the dried pre-extracted powder in water. The undissolved material was again spun down and discarded. The supernatant was adjusted to pH=7.0 by addition of 1M Tris-HCl buffer and applied on a DEAE ion-exchange column. After elution of the unbound components, the active fraction was eluted with 20 mM Tris-HCl buffer (pH=7.0) containing 500 mM NaCl.

The support for the affinity chromatography was prepared by hydrolysing Sepharose 2B in 1 M HC1 for 3h at 50°C. The ion-exchange-purified fraction was applied to a chromatography column containing the hydrolysed Sepharose. Unbound components were washed out with 20 mM Tris-HCl buffer (pH=7.0) containing 500 mM NaCl and the active fraction was eluted with 20 mM Tris-HCl buffer (pH=7.0) containing 500 mM NaCl and 100 mM galactose. Two proteins could be identified which could be partially separated from each other (Figure 3c). Both proteins have a MW of about 60 kDa and each are composed of two subunits with a MW of 26 - 32. By comparing the biological effects of various fractions having different protein compositions it was found that both proteins exhibit an anti-neoplastic effect. One of the proteins was further characterized and the corresponding gene was sequenced.

### (F) Characterization of the active compound

### (1) Analysis by mass spectroscopy

One of the purified proteins was subjected to analysis by MS-MS mass spectroscopy. The amino acid sequence of some of the MS-identified peptides was determined. It was found that three of these peptides showed homologies to a class of already known proteins, the so-called type II ribosomal inhibiting proteins (RIPs).

### (2) Sequencing

On the basis of the amino acid sequences of the peptides, degenerated primers were developed which allowed to clone a 410 bp fragment. From this sequence information, the complete cDNA was sequenced, using RNA from *Ximenia americana* of US-origin. The sequence revealed the existence of two alleles, which share more than 95% sequence identity (Figure 7) (SEQ ID NOs:3 and 4). Similarly, the incomplete coding genomic DNA sequence for riproximin was obtained from *Ximenia americana* from Tanzanian origin and showed over 94% identity to the DNA and amino acid sequence of US-origin, respectively (Figure 6(a)(SEQ ID NO:1)+(b)(SEQ ID NO:2). The translated amino acid sequence identifies the new protein, which was named riproximin, as a member of the type II RIP family. Table 2 gives a comparison of the amino acid sequence of riproximin with that of known type II RIPs. The percent similarity and identity between the amino acid sequences of riproximin and the other type II RIPs did not exceed 58% and 50%, respectively. Accordingly, a further protein and cDNA sequence of a riproximin-homolog (Rpx2) from *Ximenia americana* were obtained which are shown in Figure 11 (SEQ ID NOs:5 and 6).

Fig. 8 shows an alignment of the riproximin sequence to the database sequences of ricin (SwissProt: rici-ricco, P02879) and viscum lectin I (SwissProt: ml1_visal, P81446). At 28 positions of the riproximin cDNA sequence single nucleotide polymorphisms (SNPs) were identified, 10 of which lead to the translation of different amino acids. At the positions affected by the cDNA SNPs, both amino acids were specified. Two of the 10 amino acid variations are contained in the N-terminal signal peptide, six in the sequence of the A-chain and two in that of the B-chain.

### Sequence analysis:

For sequence analysis, programs from the HUSAR package (DKFZ, Heidelberg, Germany) were used. Data base search with newly obtained peptide/DNA sequences was performed with the program FASTA. The respective DNA sequences were assembled with the program CAP. The resulting protein sequence was aligned with other RIP sequences with the program CLUSTAL. Homology and identity to known RIP-sequences were calculated with the program GAP.

The sequence of the riproximin precursor protein shares 55% similarity and 47% identity with that of the ricin precursor as well as 53% similarity and 45% identity with the sequence of the viscum lectin I precursor, both of which belong to the subgroup of toxic type II RIPs. On the other hand, the sequence homology to the non-toxic *Sambucus* type II RIPs ebulin 1 (EMBL: SEB400822, AJ400822) and nigrin b (Swiss- Prot: NIGRB_SAMNI, P33183) was lower (48% and 45% similarity as well as 40% and 37% identity, respectively). These homology data suggest that riproximin is a toxic member of the type II RIP family. N-terminal blockade prevented determining the N-terminal sequence of the B-chains (apparent MW 34 and 31 kDa) by Edman degradation. The A-chains also showed a high extent of N-terminal blocking. However, by increasing the amount of protein, both of the A-chains (apparent MW = 27 and 29 kDa, respectively) revealed the N- terminal sequence DYP, a sequence found at position 57-59 of the cloned riproximin precursor. The probable C-terminal end of the A- and the beginning of the B-chain were estimated based on homology to other type two RIPs including ricin, viscum lectin I, abrin, cinnamomin, ebulin and nigrin, as described below. The sequence of the presumed A- and B-chains is indicated in Fig. 8. The amino acid numbering was based on the sequence of the precursor protein. The MW calculated for the riproximin A and B-chains varied between 29 and 31 kDa, pending on the exact A-chain C-terminus and B-chain N-terminus positions and the allelic variation. The presumed A-chain shows 49% similarity and 42-43% identity to that of viscum lectin I, 49-50% similarity and 41-42% identity to the ricin A-chain, and 42% similarity and 33-35% identity to that of nigrin b. The invariant residues within the active site are all conserved within the sequence of the riproximin A-chain: tyr⁷³, arg⁸¹, tyr¹³³, tyr¹⁷², glu²³⁰, arg²³³ and trp²⁶⁷ correspond to ricin tyr²¹, arg²⁹, tyr⁸⁰, tyr¹²³, glu¹⁷⁷, arg¹⁸⁰ and trp²¹¹. In addition, the conserved C-terminal cysteine residue (cys²⁵⁹ in the A-chain of ricin) which is involved in the intermolecular disulphide bonding corresponds to cys³¹⁷ in riproximin and signals the end of the riproximin A-chain. A second cysteine-residue within the A-chain, cys²²⁴, corresponds to cys¹⁷¹ in the A-chain of ricin but is not present in the viscum lectin I A-chain. While the ricin A-chain contains two glycosylation sites, the only potential glycosylation site of the riproximin A-chain was identified within the sequence of one of the alleles (asn¹⁵⁹), and is replaced by thr¹⁵⁹ in the other allele.

The riproximin B-chain shares 65% and 57-58% similarity as well as 57-58% and 49-50% identity with the B-chains of the toxic RIPs ricin and viscum lectin I, respectively. The homology to the non-toxic RIPs ebulin 1 and nigrin b is lower, as shown by 53% and 51% similarity as well as 45-46% and 43-44% identity, respectively. The eight cysteine residues known to be involved in four conserved intra-molecular disulphide-bridges in the type II RIP B-chains ( cys²⁰-cys³⁹, cys⁶³-cys⁸⁰, cys¹⁵¹-cys¹⁶⁴, cys¹⁹⁰- cys²⁰⁷ in ricin), as well as the B-chain N-terminal cysteine involved in the formation of the intermolecular disulphide-bridge between the A- and B-chains (cys⁴ in ricin), are all found in the riproximin B-chain as cys³⁵³ -cys³⁷² , cys ³⁹⁶-cys⁴¹⁵ , cys⁴⁸⁷ -cys⁵⁰⁰ , cys⁵²⁶ - cys⁵⁴⁶ (intra-molecular bridges) and cys³³⁷ (N-terminal, binding A-chain cys³¹⁷). In addition, the B-chain of riproximin contains another cysteine residue (cys⁵²⁹) aligning to cys¹⁹⁴ of the viscum lectin I B-chain. All highly conserved residues previously reported to be involved in building the hydrophobic core of the B-chain are conserved or replaced by similar hydrophobic amino acids. The riproximin B-chain contains two potential glycosylation sites, asn ⁴³⁰ and asn ⁴⁷⁰ , which correspond to highly conserved glycosylation sites within the B-chain sequence of other type II RIPs (asn⁹⁵ and asn¹³⁵ in the B-chain of ricin).

A comparison of the two sugar-affinity domains found in the B-chains of type II RIPs shows that most of the amino acid residues involved in sugar-binding are conserved in the B-chain of riproximin. The 1α subdomain amino acid residues asp²² trp³⁷, asn⁴⁶ and gln⁴⁷ of the ricin B-chain correspond to asp³⁵⁵, trp³⁷⁰, asn³⁷⁹ und gln³⁸⁰ in the 1α subdomain of the riproximin B-chain, whereas gin in ricin is replaced by ile³⁶⁸ in riproximin. The 2γ subdomain amino acid residues asp²³⁴, asn²⁵⁵ and gln²⁵⁶ of ricin correspond to asp⁵⁷³, asn⁵⁹⁴ and gln⁵⁹⁵ in the 2γ subdomain of riproximin, whereas ile²⁴⁶ and tyr²⁴⁸ in ricin are replaced by leu⁵⁸⁵ and trp⁵⁸⁷ in riproximin, respectively.

### Homology modelling:

Based on the alignment of the riproximin precursor sequence with known type II RIP-sequences, the putative sequences for the A and B riproximin chains were chosen and a FASTA-search of the PDB-database (www.pdb.com) was performed. For modelling the riproximin A-chain, the structure of the recombinant ricin R213D A-chain was chosen (PDB accession no. 1UQ4, (21)). Modelling of the riproximin B-chain was based on the structure of the mistletoe lectin I B-chain, refined to 2.05 (PDB accession no. 1SZ6). The A- and B-chain models were calculated following the approach of comparative protein modelling on the SWISS-MODEL Automated Protein Modeling Server (Schwede et al. (2003) Nucleic Acids Res.31, 3381-3385). The quality of the models was checked with the programs WHAT_CHECK (Hooft et al. (1996) Nature 381, 272) as well as PROCHECK (Pontius et al. (1996) Journal of Molecular Biology 264, 121-136).

According to the PROCHECK* analysis (Ramachandran plot), 88.6% of the A-chain- amino acids were in favored, 11.0% in allowed and only 0.4% (arg¹⁵⁵) in disallowed conformation. For the B-chain, 80.7% and 18.5% of the amino acids were in favored or allowed conformation, respectively, and only 0.8% (tyr⁴¹³, ile⁴¹⁷) was found within the disallowed area. The overall average G-Factor (ideally > -0.5) was calculated to be -0.03 for the A chain model and as -0.15 for the B chain model, respectively. The amino acid residues predicted to be involved in the ribonuclease activity of the A-chain cluster within a cleft on the surface and probably constitute the active catalytic site. The B-chain is characterized by a two-domain structure, each of them containing a potential sugar- binding site. The location of eight cysteine residues allows the formation of the four predicted intra-molecular disulphide bridges within the B-chain. The three potential glycosylation sites of riproximin are located on the surface of the molecule and thus are probably glycosylated in the mature protein. Except for asn¹⁵⁹, which is the only glycosylation site on the A-chain, none of the allelic variations of riproximin shows a significant effect on the three-dimensional structure, glycosylation, disulphide bridge formation, catalytic site or sugar binding domains.

Upon comparison with the sequences of ricin or viscum lectin I as well as after modeling the 3D-structure of the riproximin A- and B-chains it is obvious that none but one of these amino acids affected by SNP is involved in the function or structure of the protein. The only exception is the asn/thr variation at position 159, which, when expressing asn¹⁵⁹, could serve as a glycosylation site on the riproximin A-chain. Because of its singularity, the presence of this glycosylation site could contribute to a major difference between the A-chains alleles. All of the invariant amino acids involved in the N-glycosidase activity of a RIP A-chain are conserved and cluster in the 3D model within a cleft likely to be the active site of the riproximin A-chain. It can be therefore assumed that the A-chain of riproximin is a fully active RNA N-glycosidase. Similar to other type II RIP A-chains, the A-chain of riproximin has very low lysine content (two of 263 amino acid residues). The low lysine content has been shown to be involved in the RIP-cytotoxicity, since it helps the RIP A-chain to escape ubiquitination and subsequent degradation upon relocation to the cytosol (Lord et al. (2003) Biochem. Soc. Trans. 31, 1260-1262).
The B-chain of type II RIPs consists of two homologous domains termed 1 and 2, each containing three sub-domains termed 1α, 1β, 1γ and 2α, 2β, 2γ (30). The sub- domains 1α and 2γ are reported to be responsible for the sugar-affinity of the B-chains of ricin (Rutenber (1987) Nature 326, 624-626), viscum lectin I (Krauspenhaar et al. (1999) Biochem. Biophys. Res. Commun. 257, 418-424). The B-chain of riproximin also shows this typical structure, including the conserved disulphide bridges and glycosylation sites. In addition, most of the amino acids which are responsible for sugar binding in ricin and viscum lectin I, are also present in the re- spective riproximin B-chain subdomains. Surprisingly, a dynamic modeling of the sugar-binding sites revealed distinct differences to those of ricin and viscum lectin-I.

### (3) Biological activity

The proteins in the affinity-purified active fraction showed very high levels of cytotoxicity in MCF7 breast cancer cells *in vitro*, with an IC50 of 0.2 ng/ml (Figure 5). Protein concentrations and IC₅₀ values are given in ng dry protein/ml. The amount (ng) of dry protein in the affinity purified fraction was determined by lyophilisation.

### (4) Inhibition of protein synthesis

The efficacy in inhibiting the protein synthesis was determined for both native and reduced riproximin (riproximin treated with 1% β-mercaptoethanol for 1h at 37°C, to dissociate the A and B chains), obtained by affinity purification, as described under (E).

A non-radioactive method based on the TNT Quick Coupled Transcription/Translation System (Promega, Mannheim, Germany) was used to determine the protein synthesis-inhibiting activity (Langer et al.; (1996) Analytical Biochemistry 243, 150-153). Equal amounts (8 x 28.5 µl) of a transcription/translation mix (240 µl TNT Quick Master Mix, 6 µl 1 mM methionine, 12 µl T7 luciferase control DNA and 27 µl H₂O) were distributed into separate tubes (two control and six inhibition reactions) and incubated at 30°C. After 5, 7, 9, 11, 13 and 15 min incubation, 2 µl samples of the first control tube were diluted in 80 µl bioluminescence buffer (20 mM Tricin, 0.05% (w/v) BSA, pH = 7.8) and immediately frozen in liquid nitrogen to stop the reaction. Thereafter, 1.5 µl Tris-HCl buffer (20 mM Tris-HCl, pH = 7.0, control) or Tris-HCl buffer containing appropriate riproximin dilutions (inhibiton reactions) were added to the reaction mix in the remaining seven tubes and further incubated at 30°C. Samples (2 µl) were collected from each reaction at 18, 20, 22, 24, 26, 28 and 30 min, respectively, and treated as described above. The relative luciferase content of the samples was determined with the Luciferase Assay System (Promega, Mannheim, Germany) on a MicroLumat Plus luminometer (Berthold Technologies, Bad Wildbach, Germany). 5 µl of each diluted sample were mixed with 50 µl luciferase reagent and the chemoluminescence (cpm) was measured for 10 s after an initial delay of 2 s. For each reaction, the absolute luminescence count of the 18 min sample was subtracted from that of the 24 min sample (values in sqrt), yielding the relative amount of luciferase generated within this interval. The results were calculated as per cent of the luciferase amount generated in the control reaction (100%) and plotted against the riproximin concentration.

The affinity purified protein sample clearly inhibited the synthesis of luciferase in an in vitro reticulocyte lysate transcription/translation assay (Fig. 9). A clear concentration dependent translation inhibition was seen in response to 0.17 to 50 nM non-reduced or reduced protein (Fig. 9a and 9b), reflecting the activities of the heterodimeric protein and the separated A-chain, respectively. For calculating the relative luciferase amounts synthesized in each reaction, the linear phase of the kinetics was chosen (18-24 min). The IC50 for translation-inhibition was achieved at a riproximin concentration of 5.5 nM (non-reduced) or 2.6 nM (reduced). (Fig. 9c).

### Example 2

### In vivo efficacy

For determining the effect of the purified riproximin protein in a rat liver metastasis model, the rat colon cancer cell line CC531-lacZ was used (Wittmer et al.; (1999) Clin. Exp.Metastasis 17, 369-376; Saenger et al. (2004) J.Cancer Res.Clin.Oncol. 130, 203-210). In short, 4x10⁶ CC531-lacZ cells were implanted via the portal vein on day 0 into male Wag/Rij rats (Charles-River, Sulzfeld, Germany). Tumor bearing rats were treated perorally (by gavage) or intraperitoneally with the aqueous extract starting on day 1, as shown in Table 1. Three weeks later (day 21) the experiment was terminated, the liver of the animals was removed, weighed and kept at - 80°C until analysis. The number of tumor cells per liver was determined by the β-galactosidase assay (Applied Biosystems, Darmstadt, Germany) as described earlier (Wittmer et al. (1999)), by comparing to a standard curve established with a mixture of healthy liver tissue and rising numbers of tumor cells.

As shown in Table 3, the affinity purified protein sample obtained after a one-step DEAE-cellulose elution was administered to male tumor bearing Wag Rij rats via the peroral and the intraperitoneal route. No toxicity was observed following total dosages of interperitoneal. up to 10 pmol/kg and peroral up to 100 pmol/kg. Intraperitoneal administration of 0.25, 0.5 and 1 pmol/kg every second day dose-dependently reduced the increase in tumor cell number seen in untreated tumor bearing rats as indicated by T/C % (treated/control×100) ratios of 47.4, 34.9 and 22.6 (p < 0.05), respectively (Table 3). The effect on the liver weight was less prominent, with T/C % ratios of 57.5, 50.4 and 51.6, respectively. Peroral administration of 10 pmol/kg every second day was surprisingly effective: compared to the untreated controls, the mean liver weight of the treated animals was significantly lower (T/C % = 30.5, p < 0.05, Table 3) and their mean tumor cell number was reduced by two orders of magnitude (T/C % = 1.0, p <0.05, Table 3).

The mean number of CC531-lacZ tumor cells growing in the liver of Wag-Rij rats increased about 600-fold in untreated controls. This increase corresponds formally to more than 9 cell divisions and was paralleled by a more than threefold increase in mean liver weight (Wittmer et al. (1999).

After peroral treatment with the affinity purified protein sample, both the liver weight and the tumor cell number were significantly reduced when compared to the untreated tumor bearing control. The actual tumor cell number of peroral. treated rats corresponded to an 8-fold increase relative to the initial tumor cell implant, which is formally equivalent to 3 cell divisions. As observed for the extract, the intraperitoneal route was associated with a lower effect than the peroral route: A significantly reduced tumor cell number was seen in response to the highest dose only, causing > 77% tumor growth inhibition (defined as 100% - T/C%), whereas the difference in mean liver weight was not significant when compared to the control. When considering the net increase in mean liver weight, which is a more specific indicator of tumor proliferation than the raw mean liver weight, the higher inhibition expressed in percent of untreated control (>69%) reflects the results obtained from the tumor cell number within the variation of a bioassay.

The high efficacy of the peroral application is surprising, since proteins are commonly characterized by a low to very low bioavailability, which is due to degradation in the acidic stomach environment, to proteolytic enzymes as well as to a poor uptake through the intestinal wall. Accordingly, the viscum lectin I is only poorly absorbed via the gastrointestinal tract. In spite of a low systemic LD₅₀ in mice (5-10 µg/kg), dietary dosages as high as 500 mg/kg (mice) and 200 mg/kg (rats) are well tolerated (Pusztai et al. (1998) The Journal of Nutritional Biochemistry 9, 31-36; Pryme et al. (2004) Cancer Detect.Prev 28, 52-56). In line with this, cancer treatment with the recombinant rViscumin is based on sys- temic administration (Schoffski et al. (2005) European Journal of Cancer 41, 1431-1438; Schofski et al. (2004), Ann.Oncol 15, 1816-1824). Nevertheless, high doses of peroral administered viscum lectin I are associated with some systemic activity: a diet containing up to 10 mg viscum lectin I per day and mouse was effective in reducing the tumor mass of a non-Hodgkin lymphoma (Pryme et al. (2004) Cancer Detect. Prev. 28, 52-56). Notably, this dose is by more than six orders of magnitude higher than the effective peroral dose of riproximin (0.6 µg/kg administered every 2^{nd} day).

The differences in toxicity and specificity of type II RIPs are probably related to the sugar-specificity of the respective B-chains and their intracellular fate. In this context, it is interesting that only one of the two presumed sugar binding domains of the riproximin B-chain is supposed to bind galactose according to a flexible docking simulation assay.

### Example 3

### Activity in drug-resistant tumor cells over-expressing the MDR1 protein.

Drug resistance can be caused by ATP-binding-cassette (ABC)-transporters such as MDR1, a class of glycosylated membrane proteins which function as outward pumps for chemotherapeutic drugs.

### Experiment:

To address the question of the riproximin selectivity for cancer cells, cytotoxicity experiments were performed with doxorubicin-resistant CEM-ADR5000 human lymphoblastoid cells (Efferth et al. (2002) Blood Cells Mol.Dis 28(2), 160-168), which express >500-fold higher levels of the multidrug-resistance protein MDR1 in comparison to the respective sensitive cell line CCRF-CEM (German Collection for Microorganisms and Cell Cultures; Braunschweig, Germany). The activity was determined as in Example 1 (3). Surprisingly, these experiments showed a >500-fold higher riproximin-sensitivity of CEM-ADR5000 cells (Fig.10), which is in line with MDR1 being a highly glycosylated plasma membrane protein and suggest that MDR1 may act as a cellular receptor for riproximin. Therefore, the nucleic acid molecules and/or proteins of the invention can be used for the preparation of pharmaceutical compositions for treatment of a tumor being multidrug resistant to other chemotherapeutic drugs, in particular which resistance is caused by ATP-binding-cassette (ABC) transporters, e.g. MDR1 and/or which is resistant to doxorubicin.

**Table 1: Antiproliferative activity of an aqueous extract from Ximenia americana containing riproximin in 16 human and one rodent cell lines.**

| ***Cell line*** | **IC₁₀¹ *(µg*/*ml medium)*** | **IC₅₀² *(µg*/*ml medium)*** | **IC₉₀³ *(µg*/*ml medium)*** | ***IC₉₀*/*IC₁₀⁴*** |
|---|---|---|---|---|
| MCF7 | 0.6 | 1.7 | 10 | 16.7 |
| BV173 | 0.4 | 1.8 | 7 | 17.5 |
| CC531 | 0.8 | 3.3 | 12 | 15.0 |
| U87-MG | 1.0 | 9 | 100 | 100 |
| K562 | 5 | 11 | 180 | 36 |
| SKW-3 | 3.1 | 20 | 700 | 226 |
| HEp2 | 5 | 21 | 100 | 20 |
| NCl-H460 | 4 | 21 | 150 | 38 |
| PC3 | 3.5 | 26 | >1000 | >300 |
| MDA-MB231 | 5 | 33 | 100 | 20 |
| HT29 | 8 | 40 | 350 | 44 |
| U333 | 7 | 65 | 300 | 43 |
| SAOS2 | 20 | 66 | 1000 | 50 |
| LAMA84 | 10 | 90 | 600 | 60 |
| HL60 | 30 | 90 | 1000 | 33 |
| CML-T1 | 2.5 | 160 | 1000 | 400 |
| AR230 | 17 | 170 | 700 | 41 |

| | | | | |
|---|---|---|---|---|
| ¹inhibitory concentration 10 (concentration inhibiting the cell growth by 10%), as assessed by MTT-assay (Konstantinov et al., Int.J.Cancer 77 (1998), pp. 778-786). ²inhibitory concentration 50 (Concentration inhibiting the cell growth by 50%), as assessed by MTT-assay ³inhibitory concentration 90 (Concentration inhibiting the cell growth by 90%), as assessed by MTT-assay ⁴ratio of IC₉₀ and IC₁₀ values | | | | |

**Table 2: Comparison of the derived amino acid sequence of riproximin of US-origin with the published sequence of 7 other type II RIP proteins.**

| ***Type II RIP*** | ***Riproximin*** | |
|---|---|---|
| | ***% similarity¹*** | ***% identity*¹** |
| Ricin | 57.4 - 57.7 | 49.3 - 49.8 |
| RCA | 54.3 | 47.0-47.2 |
| Abrin | 49.1-49.7 | 42.9-43.5 |
| Viscumin | 52.9 - 53.2 | 45.2 - 45.3 |
| Ebulin | 48.7-48.9 | 40.9-41.1 |
| Nigrin1 | 46.2 - 46.4 | 38.2 - 38.9 |
| Cinnamomin | 54.3 - 54.7 | 46.3 - 46.6 |

| | | |
|---|---|---|
| ¹The percent similarity and identity depend on the allelic status of riproximin and are both given as range. | | |

**Table 3 : Antineoplastic effect of riproximin in the CC531-IacZ rat liver metastasis model.**

| ***Group No.*** | ***No. of animals*** | ***Treatment¹*** | | | ***Liver weight*** | | ***Net increase in liver weight³*** | | ***Tumor cell no.*/ *Liver²*** | |
|---|---|---|---|---|---|---|---|---|---|---|
| | | ***Dosage (pmol*/*kg) (pmol*/*kg)*** | ***Route*** | ***Total dose*** | **Mean ± SD (g)** | ***T*/*C*⁴%** | ***Mean (g)*** | ***T*/*C⁴%*** | ***Mean ± SD*** | ***T*/*C⁴%*** |
| 1 | 4 | Control | - | - | 26.5 ± 15.2 | 100.0 | 18.5 ±15.2 | 100.0 | 235 ± 197x10⁷ | 100.0 |
| 2 | 2⁵ | 10 | p.o. | 100 | 8.1 ± 0.1 | 30.4⁶ | 0.1 ± 0.1 | 0.3⁶ | 3 ± 2x 10⁷ | 1.0⁶ |
| 3 | 4 | 1 | i.p. | 10 | 13.7 ±10.2 | 51.6 | 5.7 ± 10.2 | 30.7 | 58 ± 99 x10⁷ | 22.6⁶ |
| 4 | 5 | 0.5 | i.p. | 5 | 13.416.6 | 50.4 | 5.4 ± 6.6 | 29.0 | 89 ± 117x10⁷ | 34.9 |
| 5 | 4 | 0.25 | i.p. | 2.5 | 15.2 ± 7.6 | 57.5 | 7.2 ± 7.6 | 39.1 | 121 ± 138 x 10⁷ | 47.4 |

| | | | | | | | | | | |
|---|---|---|---|---|---|---|---|---|---|---|
| ¹Administration started on day 1 and was repeated every second day until day 19, on day 21 the animals were sacrificed. ²4×10⁶ CC531-lacZ cells were implanted on day 0. ³The net increase in liver weight was calculated by subtracting the initial liver weight (8 g, corresponding to the mean weight of a normal rat liver) from the observed liver weight, respectively. ⁴Treated over control x 100. ⁵The small number of animals tested was due to the limited amount of protein available. ⁶p<0.05 (Wilcoxon rank sum test). | | | | | | | | | | |

## Claims

1. A nucleic acid molecule encoding the plant type II ribosome-inactivating protein (RIP) riproximin exhibiting anti-neoplastic activity, which is:
(a) a nucleic acid molecule encoding a polypeptide that comprises the amino acid sequence as depicted in Figure 7 or in Figure 11 (SEQ ID NOs:3 or 4 or 6);
(b) a nucleic acid molecule comprising the nucleotide sequence as depicted in Figure 6(a)(SEQ ID NO:1) or (b)(SEQ ID NO:2) or in Figure 11 (SEQ ID NO:5);
(c) a nucleic acid molecule encoding a polypeptide the sequence of which is at least 80% identical to the amino acid sequence of the polypeptide encoded by a nucleic acid molecule specified in (a) or (b);
(d) a nucleic acid molecule the sequence of which differs from the sequence of a nucleic acid molecule of (a) to (c) due to the degeneracy of the genetic code.

2. A recombinant vector containing the nucleic acid molecule of claim 1.

3. The recombinant vector of claim 2 wherein the nucleic acid molecule is operatively linked to regulatory elements allowing transcription and synthesis of a translatable RNA in prokaryotic and/or eukaryotic host cells.

4. A recombinant host cell which contains the recombinant vector of claim 2 or 3.

5. The recombinant host cell of claim 4, which is a bacterial cell or an eukaryotic cell, wherein the eukaryotic cell is, for example, a mammalian cell, an insect cell, a yeast cell or a plant cell.

6. A polypeptide exhibiting an anti-neoplastic activity which comprises an amino acid sequence which is at least 80% identical to one of the amino acid sequences as depicted in Fig. 7 (SEQ ID NOs:3 or 4) or in Figure 11 (SEQ ID NO:6).

7. The polypeptide according to claim 6 which is encoded by a nucleic acid molecule of claim 1.

8. A method of producing a polypeptide exhibiting an anti-neoplastic activity comprising:
(a) culturing the recombinant host cell of claim 4 or 5 under conditions such that said polypeptide is expressed; and
(b) recovering said polypeptide.

9. A polypeptide produced by the method of claim 8.

10. A conjugate comprising the polypeptide of anyone of the claims 6, 7 or 9.

11. The conjugate of claim 10 which is an immunoconjugate.

12. An antibody that binds specifically to the polypeptide of any one of the claims 6, 7 or 9.

13. The nucleic acid molecule of claim 1, the polypeptide of claim 6, 7 or 9, the conjugate of claim 10 or 11, or the antibody of claim 12 which is detectably labeled.

14. The nucleic acid molecule, the polypeptide, the conjugate, or the antibody of claim 13, wherein the label is a radioisotope, a bioluminescent compound, a chemiluminescent compound, a fluorescent compound, a metal chelate, or an enzyme.

15. A pharmaceutical composition comprising a nucleic acid molecule of claim 1, a polypeptide of claim 6, 7 or 9, a conjugate of claim 10 or 11, or a recombinant vector of any one of claims 2 to 4 and a pharmaceutically acceptable excipient, diluent or carrier.

16. Use of a nucleic acid molecule of claim 1, a polypeptide of claim 6, 7 or 9 or a plant extract containing the polypeptide of claim 6, 7 or 9, a conjugate of claim 10 or 11, or a recombinant vector of any one of claims 2 to 4 for the preparation of a pharmaceutical composition for treatment of a tumor or for immunomodulation.

17. The use of claim 16, wherein the tumor is multidrug resistant.

18. Use of a plant from the genus of *Ximenia* for obtaining a type II ribosome-inactivating protein comprising the amino acid sequence as depicted in Fig. 7 or in Fig. 11.

## Patentansprüche

1. Nukleinsäuremolekül, kodierend das Pflanzentyp II Ribosomen inaktivierende Protein (RIP) Riproximin, das antineoplastische Aktivität zeigt, welches ist:
(a) ein Nukleinsäuremolekül, das ein Polypeptid kodiert, welches die Aminosäuresequenz umfasst, wie in der Figur 7 oder in der Figur 11 gezeigt (SEQ ID NO:3 oder 4 oder 6);
(b) ein Nukleinsäuremolekül, das die Nukleotidsequenz umfasst, wie in der Figur 6(a) (SEQ ID NO:1) oder (b) (SEQ ID NO:2) oder in der Figur 11 (SEQ ID NO: 5) gezeigt;
(c) ein Nukleinsäuremolekül, das ein Polypeptid kodiert, dessen Sequenz zu mindestens 80 % mit der Aminosäuresequenz des Polypeptids identisch ist, das durch ein in (a) oder (b) angegebenes Nukleinsäuremolekül kodiert ist;
(d) ein Nukleinsäuremolekül, dessen Sequenz sich von der Sequenz eines Nukleinsäuremoleküls von (a) bis (c) aufgrund der Degeneriertheit des genetischen Kodes unterscheidet.

2. Rekombinanter Vektor, enthaltend das Nukleinsäuremolekül nach Anspruch 1.

3. Rekombinanter Vektor nach Anspruch 2, wobei das Nukleinsäuremolekül funktionsfähig mit regulatorischen Elementen verbunden ist, die die Transkription und Synthese einer translatierbaren RNA in prokaryontische und/oder eukaryontische Wirtszellen ermöglicht.

4. Rekombinante Wirtszelle, die den rekombinanten Vektor nach Anspruch 2 oder 3 enthält.

5. Rekombinante Wirtszelle nach Anspruch 4, die eine bakterielle Zelle oder eine eukaryontische Zelle ist, wobei die eukaryontische Zelle zum Beispiel eine Säugetierzelle, eine Insektenzelle, eine Hefezelle oder eine Pflanzenzelle ist.

6. Polypeptid mit antineoplastischer Aktivität, das eine Aminosäuresequenz umfasst, die zu mindestens 80 % identisch mit den Aminosäuresequenzen ist, wie in der Figur 7 (SEQ ID NO:3 oder 4) oder in der Figur 11 (SEQ ID NO:6) gezeigt.

7. Polypeptid nach Anspruch 6, welches durch ein Nukleinsäuremolekül nach Anspruch 1 kodiert ist.

8. Verfahren zur Herstellung eines Polypeptids, das eine antineoplastische Aktivität zeigt, umfassend:
(a) das Kultivieren der rekombinanten Wirtszelle nach Anspruch 4 oder 5 unter solchen Bedingungen, dass das Polypeptid exprimiert wird; und
(b) das Gewinnen des Polypeptids.

9. Polypeptid, erzeugt durch das Verfahren nach Anspruch 8.

10. Konjugat, umfassend das Polypeptid nach einem der Ansprüche 6, 7 oder 9.

11. Konjugat nach Anspruch 10, das ein Immunkonjugat ist.

12. Antikörper, der speziell an das Polypeptid nach einem der Ansprüche 6, 7 oder 9 bindet.

13. Nukleinsäuremolekül nach Anspruch 1, Polypeptid nach Anspruch 6, 7 oder 9, Konjugat nach Anspruch 10 oder 11, oder Antikörper nach Anspruch 12 mit nachweisbarer Markierung.

14. Nukleinsäuremolekül, Polypeptid, Konjugat oder Antikörper nach Anspruch 13, wobei die Markierung ein Radioisotop, eine biolumineszierende Verbindung, eine chemilumineszierende Verbindung, eine fluoreszierende Verbindung, ein Metallchelat oder ein Enzym ist.

15. Pharmazeutische Zusammensetzung, umfassend ein Nukleinsäuremolekül nach Anspruch 1, ein Polypeptid nach Anspruch 6, 7 oder 9, ein Konjugat nach Anspruch 10 oder 11 oder einen rekombinanten Vektor nach einem der Ansprüche 2 bis 4 und einen pharmazeutisch annehmbaren Hilfsstoff, Verdünner oder Träger.

16. Verwendung eines Nukleinsäuremoleküls nach Anspruch 1, eines Polypeptids nach Anspruch 6, 7 oder 9 oder eines Pflanzenextrakts, enthaltend das Polypeptid nach Anspruch 6, 7 oder 9, eines Konjugats nach Anspruch 10 oder 11 oder eines rekombinanten Vektors nach einem der Ansprüche 2 bis 4 zur Herstellung einer pharmazeutischen Zusammensetzung zur Behandlung eines Tumors oder zur Immunmodulation.

17. Verwendung nach Anspruch 16, wobei der Tumor Multidrugresistenz hat.

18. Verwendung einer Pflanze der Gattung Ximenia zum Gewinnen eines Ribosomen inaktivierenden Proteins vom Typ II, umfassend die Aminosäuresequenz, wie in der Figur 7 oder in der Figur 11 gezeigt.

## Revendications

1. Molécule d'acide nucléique encodant la protéine végétale d'inactivation du ribosome de type II (RIP) riproximine montrant une activité anti-néoplastique qui est:
(a) une molécule d'acide nucléique encodant un polypeptide qui comprend la séquence d'acides aminés telle que décrite sur la figure 7 ou sur la figure 11 (SEQ ID N°3 ou 4 ou 6),
(b) une molécule d'acide nucléique comprenant la séquence nucléotidique telle que décrite sur la figure 6(a) (SEQ ID N°1) ou (b) (SEQ ID N°2) ou sur la figure 11 (SEQ ID N° 5),
(c) une molécule d'acide nucléique encodant un polypeptide dont la séquence est identique à au moins 80% à la séquence d'acides aminés du polypeptide encodé par une molécule d'acide nucléique spécifiées dans (a) ou (b),
(d) une molécule d'acide nucléique dont la séquence diffère de la séquence d'une molécule d'acide nucléique selon (a) à (c) à cause de la dégénérescence du code génétique.

2. Vecteur recombinant contenant la molécule d'acide nucléique selon la revendication 1.

3. Vecteur recombinant selon la revendication 2, **caractérisé en ce que** la molécule d'acide nucléique est liée de manière fonctionnelle aux éléments de régulation permettant la transcription et la synthèse d'un ARN traduisible dans des cellules hôtes procaryotes et/ou eucaryotes.

4. Cellule hôte recombinante qui contient le vecteur recombinant selon la revendication 2 ou 3.

5. Cellule hôte recombinante selon la revendication 4, qui est une cellule bactérienne ou une cellule eucaryote, **caractérisée en ce que** la cellule eucaryote est, par exemple, une cellule de mammifère, une cellule d'insecte, une cellule de levure ou une cellule végétale.

6. Polypeptide montrant une activité anti-néoplastique qui comprend une séquence d'acides aminés qui est identique à au moins 80% à une des séquences d'acides aminés telles que décrites sur la figure 7 (SEQ ID N°3 ou 4) ou sur la figure 11 (SEQ ID N°6).

7. Polypeptide selon la revendication 6 qui est encodé par une molécule d'acide nucléique selon la revendication 1.

8. Méthode de production d'un polypeptide montrant une activité anti-néoplastique comprenant :
(a) cultiver la cellule hôte recombinante selon la revendication 4 ou 5 dans des conditions telles que le dit polypeptide est exprimé, et
(b) récupérer le dit polypeptide.

9. Polypeptide produit par la méthode selon la revendication 8.

10. Conjugué comprenant le polypeptide selon l'une quelconque des revendications 6, 7 ou 9.

11. Conjugué selon la revendication 10 qui est un conjugué immunologique.

12. Anticorps qui se lie spécifiquement au polypeptide selon l'une quelconque des revendications 6, 7 ou 9.

13. Molécule d'acide nucléique selon la revendication 1, polypeptide selon la revendication 6, 7 ou 9, conjugué selon la revendication 10 ou 11, ou anticorps selon la revendication 12 qui est étiqueté de manière détectable.

14. Molécule d'acide nucléique, polypeptide, conjugué, ou anticorps selon la revendication 13, **caractérisé en ce que** l'étiquette est un isotope radioactif, un composé bioluminescent, un composé chimio-luminescent, un composé fluorescent, un chélate métallique ou une enzyme.

15. Composition pharmaceutique comprenant une molécule d'acide nucléique selon la revendication 1, un polypeptide selon la revendication 6, 7 ou 9, un conjugué selon la revendication 10 ou 11 ou un vecteur recombinant selon l'une quelconque des revendications 2 à 4 et un excipient, diluant ou porteur acceptable pharmaceutiquement.

16. Utilisation d'une molécule d'acide nucléique selon la revendication 1, d'un polypeptide selon la revendication 6, 7 ou 9 ou d'un extrait de plante contenant le polypeptide selon la revendication 6, 7 ou 9, d'un conjugué selon la revendication 10 ou 11 ou d'un vecteur recombinant selon l'une quelconque des revendications 2 à 4 pour la préparation d'une composition pharmaceutique pour le traitement d'une tumeur ou pour la modulation immunitaire.

17. Utilisation selon la revendication 16, **caractérisée en ce que** la tumeur est multi-résistante aux médicaments.

18. Utilisation d'une plante du genre *Ximenia* pour obtenir une protéine d'inactivation du ribosome de type II comprenant la séquence d'acides aminés telle que décrite sur la figure 7 ou sur la figure 11.
